# EUROPEAN PATENT APPLICATION

(11) **EP 3 718 516 A2**
(43) Date of publication of application: **07.10.2020**
(21) Application number: 20168119.4
(22) Date of filing: 04.04.2020
(51) Int. Cl.: A61F 5/08

(54) **NASAL DILATORS WITH IMPROVED BREATHABILITY**

(30) Priority: 06.04.2019 US 201916377196; 06.04.2019 US 201916377198
(71) Applicant: Ierulli, Joseph, Sarasota FL 34235 (US)
(72) Inventor: Ierulli, Joseph, Sarasota FL 34235 (US)
(74) Representative: Dickerson, David

(57) **Abstract**

Nasal dilators with improved comfort are formed as a laminate of layers: a resilient layer including a plurality of resilient members; and an engagement layer including one or both of a base layer and a cover layer. A portion of the dilator occupied by all of the resilient members plus the gap(s) between the resilient members has a total area, and the proportion of that area that is occupied or covered by the resilient members does not exceed 57%. Preferably, the proportion is lower than about 50%, and it may be as low as 30% in some embodiments.

## Description

### BACKGROUND OF THE DISCLOSURE

### FIELD OF THE DISCLOSURE

The invention relates to nasal dilators. More specifically, the invention relates to nasal-dilator configurations that provide improved wearing comfort.

More generally, the present invention relates to medical devices, and more particularly to apparatus for and methods of supporting or dilating external tissue in humans. As disclosed and taught in the preferred embodiments, the tissue dilator devices are particularly suitable for, and are directed primarily to, external nasal dilators for supporting, stabilizing, and dilating nasal outer wall tissues adjacent and overlying nasal airway passages of the human nose.

### DESCRIPTION OF THE RELATED ART

Dilators to improve breathing by expanding a person's nasal passages have been investigated and developed for many decades. One of the earliest external nasal dilator devices is described in U.S. Patent No. 1,292,083 to Sawyer, and a simple forerunner of contemporary external nasal dilators was described in Spanish utility model ES_289-561 in 1985.

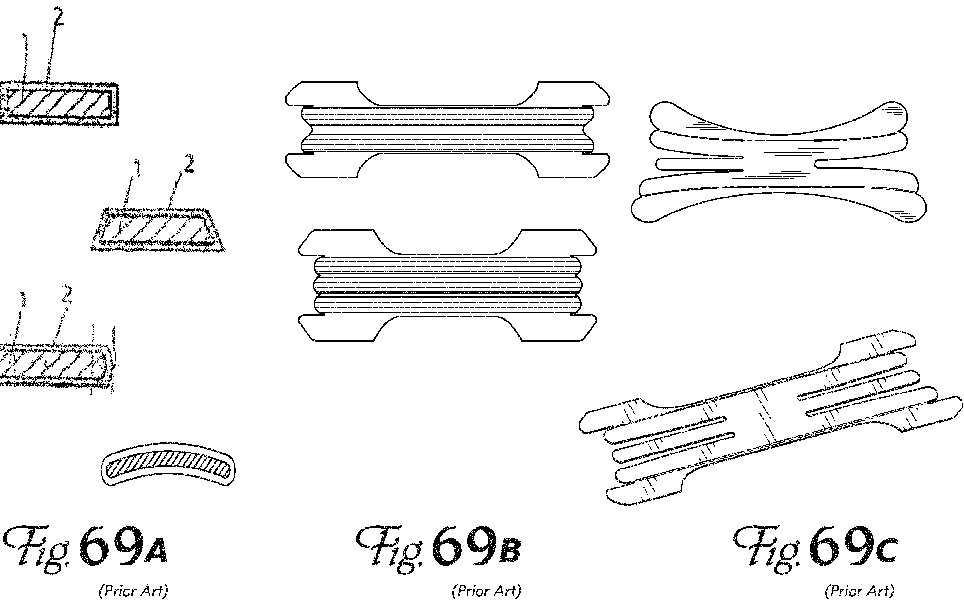

With the development of modern materials (including nonwoven fabrics, breathable films, and sturdy, lightweight resilient plastic sheet materials), most recent dilators have adopted one of three basic forms, shown in Figures 69a-c. The simplest is a "hot dog" shape (Fig. 69a): a single resilient band (which may be straight or curved) is "island-placed" within the periphery of an adhesive material. Slightly more complex is the "dog bone" shape (Fig. 69b), which includes enlarged end regions comprising corner tabs, or wings, to improve adherence, and so that the resilient members may extend fully from end to end. Dog-bone dilators typically have multiple parallel resilient members, and these are often constrained along their lengths, or at least at their ends, so as to be securely integrated into a truss-like configuration. Finally, "butterfly" dilators (Fig. 69c) have multiple separate spring fingers extending outward from a common center, intending to provide dilation at discrete points about the nose. Butterfly dilators may be distinguished from dog-bone dilators in part by the fact that the lateral ends of the resilient member(s) are not constrained relative to each other; their four (or more) resilient branches are relatively free to move independently.

Note that some prior-art dilators blur the boundaries between "hot dog," "dog bone" and "butterfly." For example, U.S. Patent No. 5,533,499 to Johnson discloses a dilator whose overall shape is that of a dog bone, but whose resilient members terminate short of the dilator ends, so they are island-placed.

All contemporary dilators must address several common challenges: first, they must adhere securely enough to avoid peeling off inadvertently, yet not adhere so aggressively that the user's skin is damaged when they are removed intentionally. Second, they must be comfortable for long-term (often overnight) wear. And finally, they must provide a consistent, reliable spring force to stabilize the skin and tissue over the user's nasal valve, and thereby to help open the user's nasal passages for improved respiration.

One occurrence that adversely impacts user comfort is the accumulation of moisture under the dilator - the skin to which the dilator is adhered may transpire or sweat, and the combination of perspiration and adhesive may cause itching. In some embodiments, adhesive-free areas or even absorbent pads are used to reduce the area where itching may occur. Other designs use special materials with higher moisture vapor transmission rates (MVTR). However, these materials are often more expensive than traditional materials, or are harder to work with.

Alternative approaches to reducing moisture-trapping surface area in a nasal dilator may be of substantial value in this area of technology.

External nasal dilators (END) are well disclosed in the art and are widely available in retail consumer markets where they are generally referred to as nasal strips or nasal dilator strips. In use the END extends over the nose, flexed across the bridge and adhered to the skin surfaces on either side thereof.

The functional part of the END is at least one resilient member (synonymously referred to in the art as a spring, spring member, resilient band, resilient member band, spring band, or bridge) that extends along the length of the nasal dilator. When flexed, the resilient member exerts spring biasing forces that urge nasal passage outer wall tissues outward, stabilizing the outer walls and expanding, or dilating, the nasal passages underneath.

Stabilized nasal outer walls and/or dilated nasal passages increase nasal patency and reduce nasal airflow resistance, beneficially affecting nasal obstruction and nasal congestion primarily about the nasal valve and extending to the nostril opening. Stabilized nasal outer walls are less likely to collapse during inhalation. Dilated nasal passages have increased cross sectional area and greater nasal cavity volume. Stabilization and/or dilation, particularly at the nasal valve, results in a corresponding improvement in nasal airflow, which may have beneficial effects generally, may increase oxygen uptake, may improve sleep, may reduce sleep disturbances, or may improve nasal snoring or obstructive sleep apnea (OSA). External nasal dilators have been shown to have beneficial effects for athletes, particularly in sports where a mouth-guard is worn. For aerobic sports, nasal strips may delay onset of intra-oral breathing, and may create a subjective feeling of increased respiration, which may provide a psychological benefit in competition.

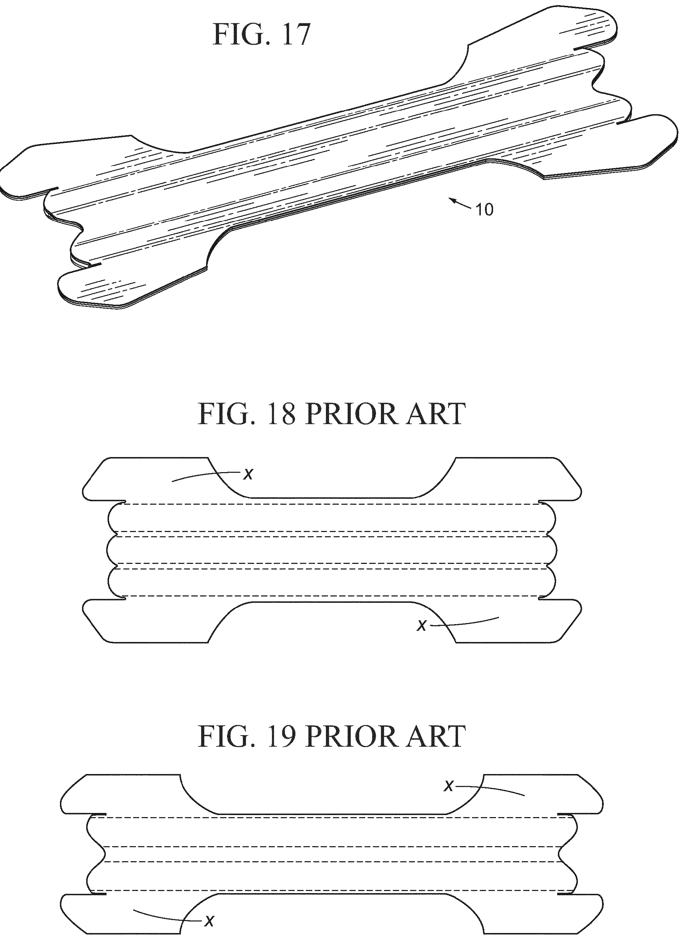

The most prevalent, common and widely available nasal dilators have two or three closely parallel resilient bands extending from end to end of the nasal dilator, and four corner tabs, *x*, as illustrated in Figs. 18 and 19. They have historically accounted for the vast majority of all external nasal dilators sold in the U.S. consumer market since introduced to consumers in 1995, according to retail sales data as provided, for example, by the Nielsen Company, US, LLC retail measurement services. These nasal dilators are efficient in terms of size, shape, resiliency, efficacy and reliability. Their construction includes a relationship between the surface area that adheres the dilator to the nose and the surface area that acts to urge the nasal passage tissues outward.

Roughly half of all nasal strips currently sold consumers in the U.S. retail market are constructed using clear polyethylene (PE) film (also according to Nielsen data) to engage the nasal dilator to the nose; the other half use a nonwoven fabric. PE film is a moisture vapor barrier. That is, the film does not 'breathe', or allow moisture vapor - as from the skin of the nose of a user - to pass through its thickness. Additionally, the nasal dilator's plastic resilient member is also impermeable to moisture and air, so 100% of the PE nasal dilator surface area is non-breathable. Where nonwoven fabrics are used instead of PE, only about half of the surface area is breathable, primarily at corner tabs x, as seen in Figs. 18 and 19.

Buildup of moisture vapor between the nasal dilator and the skin surface of the nose can cause itching and discomfort. Moisture vapor buildup may also cause adhesive failure and thus premature disengagement of the dilator from the skin surface of the nose. Premature disengagement is believed to be the most common complaint from nasal dilator users. However, for users with sensitive skin it is believed that this same buildup of moisture makes a PE film dilator easier to remove and less likely to irritate the skin upon removal. Irritation upon removal is believed to be the second most common user complaint.

Accordingly, there is an unmet need in the nasal strip market, particularly in view of the popularity of PE film nasal dilators, to provide breathable film-based nasal dilators, and to provide nasal dilators with greater breathable surface area while retaining the efficiency of current widely available nasal dilators.

Human skin surfaces have maximum breathability when not covered by an article, such as clothing or a medical device, or by a substance such as lotion or sunscreen. Adhesive articles made of breathable thermoplastic film are generally regarded as being more effective and comfortable on the skin than non-breathable film articles. Breathable films may include those that have an inherent Moisture Vapor Transmission Rate (MVTR), such as polyurethane (PU) film, or may include those that have an engineered MVTR, such as micro-porosity, made by perforating a film's thickness.

Several external nasal dilator prior art references teach that PU films are suitable nasal dilator material choices. However, nasal dilators constructed using PU film have not been heretofore available to consumers. Nasal dilators having a microporous PE film were briefly, but widely, available to U.S. consumers from about 1995 to about 1998.

The present invention provides new and nonobvious external nasal dilators that address unmet needs, that are commercially viable, and which may be mass produced on an economic scale.

### SUMMARY OF THE PRESENT DISCLOSURE

Embodiments of the invention are dog-bone style nasal dilators whose resilient members are positioned differently from the prior art, so that the breathable surface area of the dilator within the resilient structure's boundaries is substantially increased.

Nasal dilators of the present invention have a significantly greater engagement element surface area relative to the resilient element surface area compared to prior art nasal dilators. That greater surface area may be made breathable so as to increase the nasal dilator's moisture vapor transmission rate (MVTR). Greater MVTR generally makes an article worn on the skin for any length of time more comfortable.

Nasal dilators of the present invention comprise an engagement element for securing the nasal dilator to the skin surfaces of a nose of a user, and a resilient element that provides resiliency, or spring biasing forces, for stabilizing or dilating the nasal passages. The engagement element defines a plan view surface area of the nasal dilator that is substantially the same as the most widely available prior art nasal dilators. The resilient element provides the same resiliency or spring biasing force as the prior art nasal dilators, but within a substantially lesser surface area.

Some embodiments include at least one of an opening formed in the engagement element whereby MVTR may be further improved.

### BRIEF DESCRIPTION OF THE DRAWINGS

- Fig. 1: shows a perspective view of a nasal dilator, in accordance with the present invention, seen in use on a nose of a user.
- Fig. 2: shows a three-quarter perspective view of the nasal dilator illustrated in Fig. 1.
- Fig. 3: shows a top plan view of the nasal dilator of Fig. 1, the drawing including dark parallel lines to illustrate defined regions of the nasal dilator.
- Fig. 4: shows a perspective view of a nasal dilator, in accordance with the present invention, seen in use on a nose of a user.
- Fig. 5: shows a top plan view of the nasal dilator of Fig. 4.
- Fig. 6: shows an exploded perspective view of a nasal dilator in accordance with the present invention.
- Fig. 7: shows an exploded perspective view of a nasal dilator in accordance with the present invention.
- Fig. 8: shows an exploded perspective view of a nasal dilator in accordance with the present invention.
- Fig. 9: shows a three-quarter perspective view of the nasal dilator of Fig. 5.
- Fig. 10: shows an exploded perspective view of a nasal dilator in accordance with the present invention.
- Fig. 11: shows an exploded perspective view of a nasal dilator in accordance with the present invention
- Fig. 12: shows a top plan view of a nasal dilator in accordance with the present invention.
- Fig. 13: shows a perspective view of the nasal dilator of Fig. 12, seen in use on a nose of a user.
- Fig. 14: shows a three-quarter perspective view of the nasal dilator of Fig. 12.
- Fig. 15: shows a perspective view of a nasal dilator, in accordance with the present invention, seen in use on a nose of a user.
- Fig. 16: shows a top plan view of the nasal dilator of Fig. 15.
- Fig. 17: shows a three-quarter perspective view of the nasal dilator of Fig. 15.
- Fig. 18: shows a top plan view of a three-band prior art nasal dilator.
- Fig. 19: shows a top plan view of a two-band prior art nasal dilator.
- Fig. 20: shows a top plan view of the periphery of the nasal dilators depicted in Figs. 1-11 overlaid onto a top plan view of the periphery of the prior art nasal dilator depicted in Fig. 18.
- Fig. 21: shows a top plan view of the periphery of the nasal dilators depicted in Figs. 12-17 overlaid onto a top plan view of the periphery of the prior art nasal dilator depicted in Fig. 19.
- Fig. 22: shows fragmentary plan views of nasal dilators of the present invention aligned to fragmentary plan views of the prior art nasal dilators of Figs. 18 and 19.
- Fig. 23: shows a fragmentary plan view of the nasal dilator of Figs. 12-17, taken on an enlarged scale.
- Fig. 24: shows a fragmentary plan view of the nasal dilator of Figs. 1-11, taken on an enlarged scale.
- Fig. 25: shows a fragmentary plan view of the prior art nasal dilator of Fig. 18, taken on an enlarged scale.
- Fig. 26: shows a fragmentary plan view of the nasal dilator of Figs. 1-11, taken on an enlarged scale.
- Fig. 27: shows a fragmentary plan view of the prior art nasal dilator of Fig. 19, taken on an enlarged scale.
- Fig. 28: shows a fragmentary plan view of the nasal dilator of Figs. 12-17, taken on an enlarged scale.
- Fig. 29: shows a top plan view of a nasal dilator in accordance with the present invention.
- Fig. 30: shows a top plan view of a nasal dilator in accordance with the present invention.
- Fig. 31: shows a fragmentary plan view of the nasal dilator of Figs. 29-30, taken on an enlarged scale.
- Fig. 32: shows a three-quarter perspective view of the nasal dilator of Fig. 29.
- Fig. 33: shows a perspective view of the nasal dilator of Fig. 29, seen in use on a nose of a user.
- Fig. 34: shows a three-quarter perspective view of the nasal dilator of Fig. 30.
- Fig. 35: shows a perspective view of the nasal dilator of Fig. 30, seen in use on a nose of a user.
- Fig. 36: shows a top plan view of a nasal dilator in accordance with the present invention.
- Fig. 37: shows a top plan view of a nasal dilator in accordance with the present invention.
- Fig. 38: shows a fragmentary plan view of the nasal dilator of Figs. 36-37, taken on an enlarged scale.
- Fig. 39: shows a three-quarter perspective view of the nasal dilator of Fig. 36.
- Fig. 40: shows a three-quarter perspective view of the nasal dilator of Fig. 37.
- Fig. 41: shows a perspective view of the nasal dilator of Fig. 37, seen in use on a nose of a user.
- Fig. 42: shows a perspective view of the nasal dilator of Fig. 36, seen in use on a nose of a user.
- Fig. 43: shows a top plan view of a nasal dilator in accordance with the present invention.
- Fig. 44: shows a three-quarter perspective view of the nasal dilator of Fig. 43.
- Fig. 45: shows a perspective view of the nasal dilator of Fig. 43, seen in use on a nose of a user.
- Fig. 46: shows a top plan view of a nasal dilator in accordance with the present invention.
- Fig. 47: shows a perspective view of the nasal dilator of Fig. 46, seen in use on a nose of a user.
- Fig. 48: shows a three-quarter perspective view of the nasal dilator of Fig. 46.
- Fig. 49: shows a top plan view of a nasal dilator in accordance with the present invention.
- Fig. 50: shows a top plan view of a nasal dilator in accordance with the present invention.
- Fig. 51: shows a three-quarter perspective view of the nasal dilator of Fig. 49.
- Fig. 52: shows a perspective view of the nasal dilator of Fig. 49, seen in use on a nose of a user.
- Fig. 53: shows a three-quarter perspective view of the nasal dilator of Fig. 50.
- Fig. 54: shows a perspective view of the nasal dilator of Fig. 50, seen in use on a nose of a user.
- Fig. 55: shows a top plan view of a nasal dilator in accordance with the present invention.
- Fig. 56: shows a top plan view of a nasal dilator in accordance with the present invention.
- Fig. 57: shows a perspective view of the nasal dilator of Fig. 55, seen in use on a nose of a user.
- Fig. 58: shows a three-quarter perspective view of the nasal dilator of Fig. 55.
- Fig. 59: shows a perspective view of the nasal dilator of Fig. 56, seen in use on a nose of a user.
- Fig. 60: shows a three-quarter perspective view of the nasal dilator of Fig. 56.

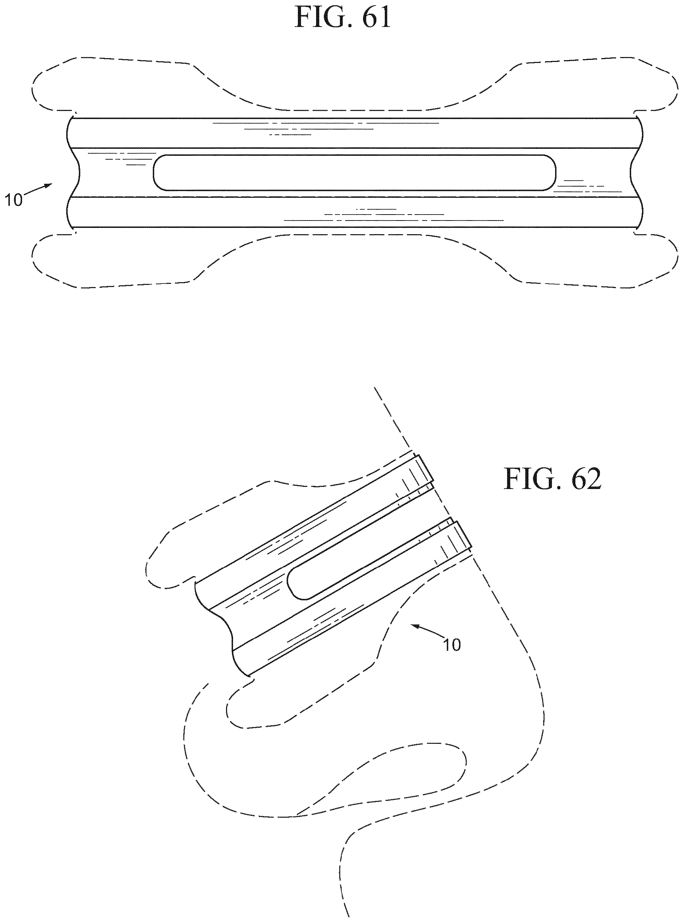

- Fig. 61: shows a top plan view of a nasal dilator in accordance with the present invention.
- Fig. 62: shows a perspective view of the nasal dilator of Fig. 61, seen in use on a nose of a user.

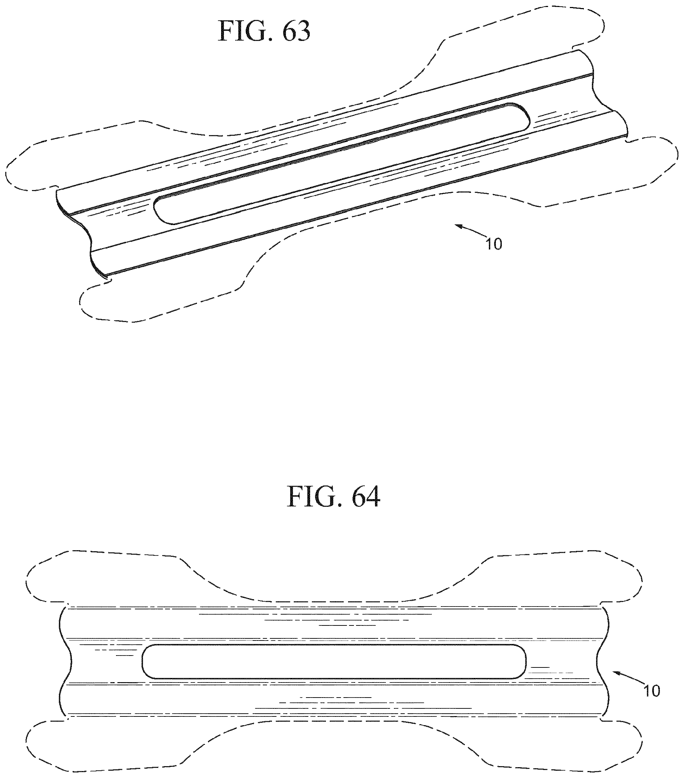

- Fig. 63: shows a three-quarter perspective view of the nasal dilator of Fig. 61.
- Fig. 64: shows a top plan view of a nasal dilator in accordance with the present invention.

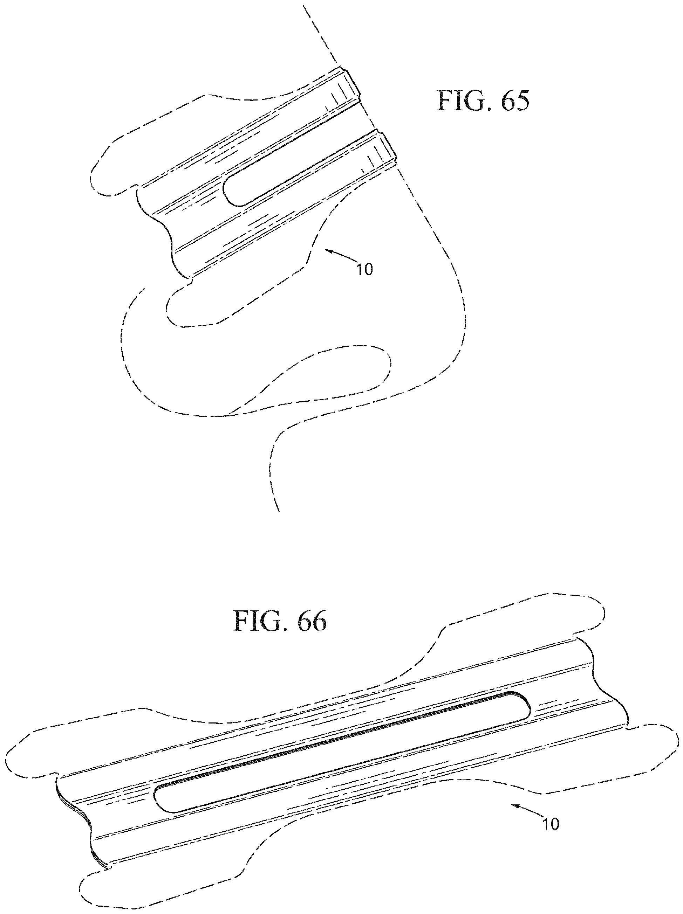

- Fig. 65: shows a perspective view of the nasal dilator of Fig. 64, seen in use on a nose of a user.
- Fig. 66: shows a three-quarter perspective view of the nasal dilator of Fig. 64.
- Figure 67: shows a representative embodiment of the invention.
- Figure 68: highlights important differences between embodiments and prior-art dog-bone dilators.
- Figures 69a-69c: show examples of prior-art dilators of the three major categories.
- Figures 70a-70d: show alternative material stacking orders in a laminated dilator according to an embodiment.
- Figure 71: shows another embodiment of the invention.
- Figure 72: shows another embodiment of the invention.
- Figure 73: shows another embodiment of the invention.
- Figures 74a-74c: show a protocol for measuring the resilient or spring force of a nasal dilator.

### DETAILED DESCRIPTION

Before discussing the embodiments shown in the Figures, the following paragraphs first elucidate the terminology used in the present disclosure and the conventions adopted in the Figures.

The terms *spring biasing, spring biasing force, spring force, resiliency, spring constant,* etc. as used herein are generally synonymous. Nasal dilators of the present invention may generate spring biasing force in a range of from about 5 grams to about 60 grams. A preferred range is from about 15 grams to about 35 grams for non-athletes, and from about 25 grams to about 45 grams for use in training, conditioning and competition by athletes. Less than 15 grams of spring biasing may not provide enough stabilization or dilation for some users, while greater than 35 grams may be uncomfortable for nonathletic use, such as during sleep, work or study.

The nasal dilator resilient member is semi-rigid; it is flexible out-of-plane with very little or no in-plane elongation. Strictly speaking, the term *resilient* may be used to describe objects that exhibit either 'flexure' or 'elasticity'. For purposes of the present invention, however, the terms *resilient, resiliency, spring biasing,* etc., mean flexure out-of-plane, in a direction perpendicular or oblique to the surface plane, while being substantially rigid in-plane. This is different from, for example, an elastic web that stretches in a direction parallel to its surface plane, even though both the elastic web and the nasal dilator resilient member may return at least substantially to their initial positions after *stretch* or *flexure,* respectively. Nasal dilators herein may be described as "capable of flexing" (when the dilator is in an initial or un-flexed position), or "flexed" (when the dilator is engaged to the nose of a user).

The present invention is not limited to the illustrated or described embodiments, which are examples of forms of the present invention. All structures and methods that embody similar functionality are intended to be covered hereby. The nasal dilators depicted, taught, enabled and disclosed herein represent new, useful and non-obvious nasal dilator devices having a variety of alternative embodiments. Some embodiments of the present invention may refer to, or cross reference, other embodiments. It may be apparent to one of ordinary skill in the art that nasal dilator features, construction or configuration may be applied, interchanged or combined between and among the preferred embodiments.

For descriptive clarity, certain terms may be used in the specification and claims: *Vertical* refers to a direction parallel to thickness, such as the thickness of a finished article, a member or component, or a laminate. *Horizontal* refers to length or longitudinal extent, such as that of a finished article or element thereof, or a direction parallel thereto. *Lateral* refers to width or lateral extent. *Longitudinal* also refers to length, perpendicular to width or lateral extent. A *longitudinal centerline* is consistent with the long axis of a finished device, element, member or layer, bisecting its width midway between the long edges. A *lateral centerline* bisects the long axis midway along its length, perpendicular to the longitudinal centerline. The terms *upper* and *lower* refer to object orientation, particularly in plan views, relative to the top and bottom of the drawing sheet.

Broken lines and dashed lines may be used in the drawings to aid in describing relationships or circumstances with regard to objects:
- A broken line including a dash followed by three short spaces with two short dashes therebetween indicates separation for illustrative purposes, such as in an exploded view, or to indicate an object or objects removed or separated from one or more other objects, primarily for illustrative clarity.
- A dashed line (sometimes referred to as a shadow line) of successive short dashes with short spaces therebetween may be used to illustrate an object or element generically, to illustrate one object underneath another, or to reference environment such as facial features; or for clarity, to show location, such as the space an object or structure will occupy, would occupy, did occupy or may occupy; or for illustrative purposes, to represent an object, structure, element or layer(s) as transparent so that other objects more pertinent to the discussion at hand may be highlighted or more clearly seen.
- A broken line including a long dash followed by a short space, a short dash and another short space is used to call out a centerline or an angle, or to indicate alignment; when accompanied by a bracket, to call out a section, segment or portion of an object or a group of objects; to illustrate a spatial relationship between one or more objects or groups of objects, or to create separation between objects for the purpose of illustrative clarity.

In the drawings accompanying this disclosure like objects are generally referred to with common reference numerals or characters, except where variations of otherwise like objects must be distinguished from one another. Where there is a plurality of like objects in a single drawing figure corresponding to the same reference numeral or character, only a portion of said like objects may be identified. After initial description in the text, some reference characters may be placed in a subsequent drawing(s) in anticipation of a need to call repeated attention to the referenced object. Where a feature or element has been previously described, shadow lines (dashed lines) may be used to generically illustrate the feature or element. Drawings are rendered to scale, but may be enlarged from actual size for illustrative clarity. Thickness may be slightly exaggerated in perspective views for illustrative clarity.

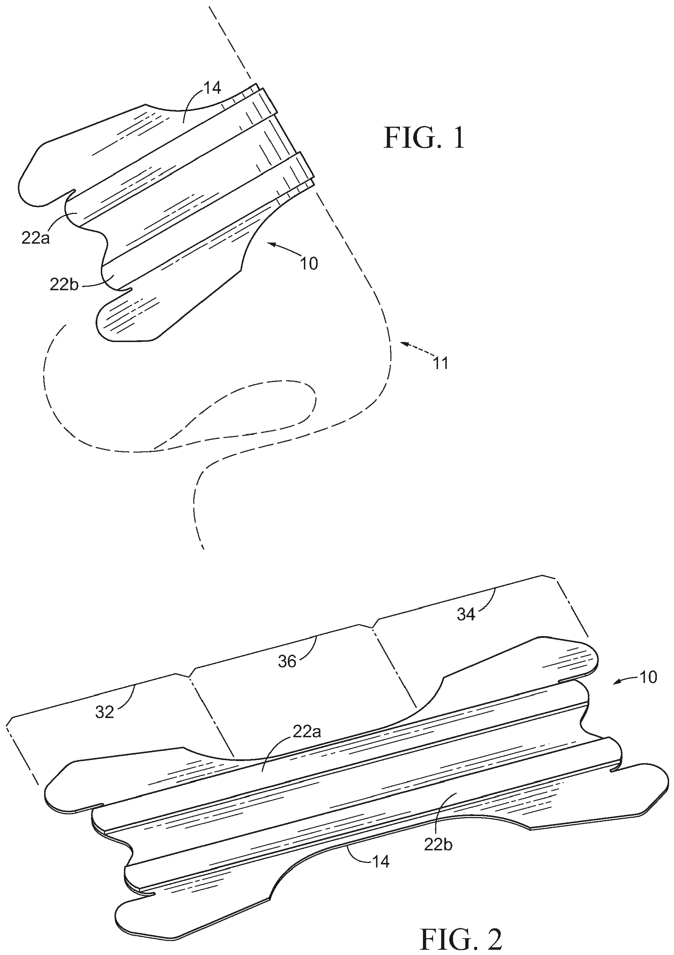

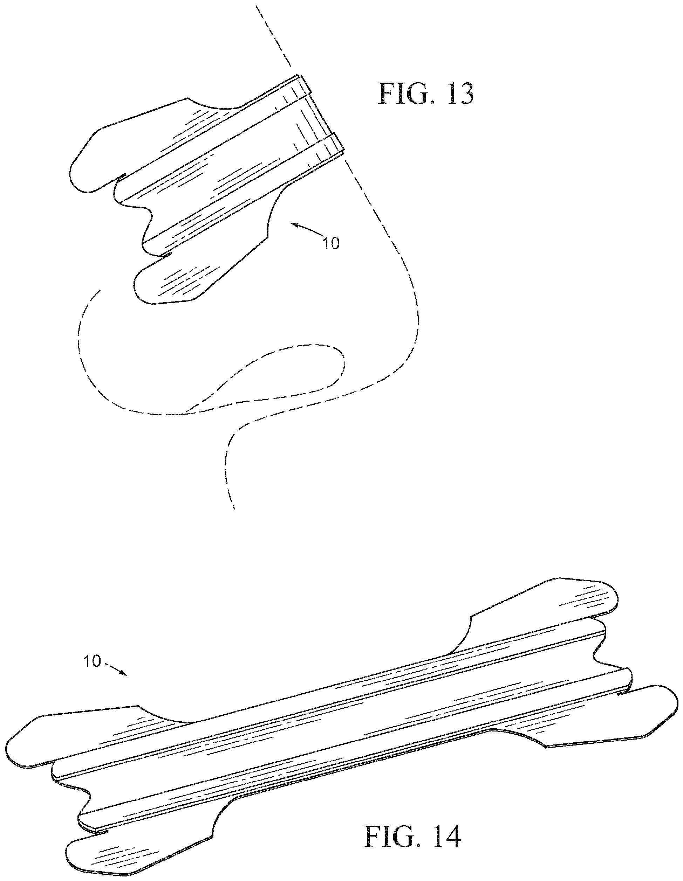

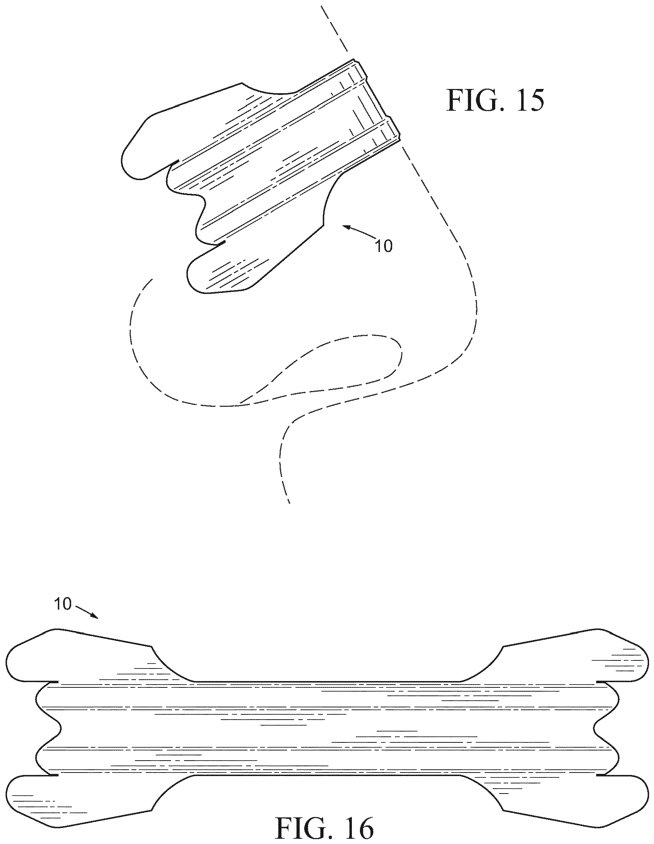

As particularly seen in Figs. 1-17 a nasal dilator, 10, comprises a laminate of vertically stacked material layers that may include: a base layer comprising at least one base member, 14 (if a plurality, 14a, 14b, etc.); a resilient layer most preferably comprising two resilient members 22a and 22b; a cover layer comprising at least one cover member, 18. A protective layer of release liner, not shown, removably covers exposed adhesive from dilator 10 preliminary to application to nose 11.

Dilator layers may be secured to each other by any suitable means such as stitching or fastening, heat or pressure bonding, ultrasonic welding, or the like, but are preferably laminated by an adhesive substance disposed on at least one flat surface side of at least one layer.

The plan view periphery and total surface area of dilator 10 may be defined by either the base layer or cover layer. The base and cover layers, either individually or combined, together with a biocompatible adhesive disposed thereon for engaging the skin, provide the engagement element of dilator 10.

As seen in Fig. 2, the vertically stacked layers of dilator 10 form a unitary, or single body, truss. Brackets and broken lines delineate approximate boundaries between regions that extend laterally across the width of dilator 10. These regions include a first end region, 32, a second end region, 34, and an intermediate region, 36.

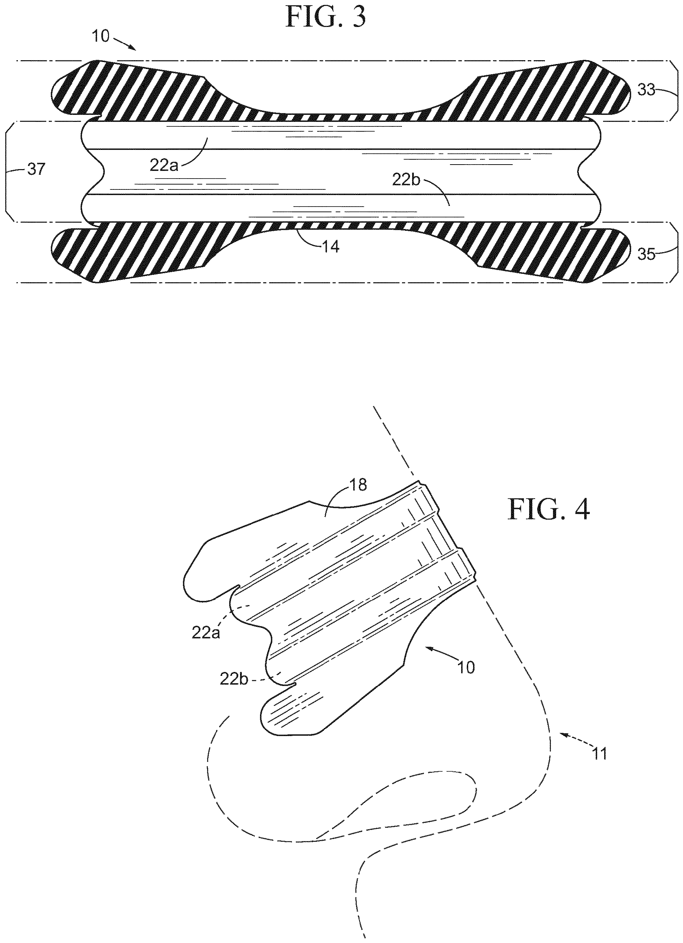

In Fig. 3, brackets and broken lines delineate specific boundaries between regions that extend fully along the length of dilator 10, including a first corner tab region, 33, a second corner tab region, 35, and a body region, 37. For illustrative emphasis the surface areas of corner tab regions 33 and 35 are marked by parallel thick dark lines. Corner tab regions 33 and 35 are positioned immediately adjacent each long edge of body region 37, abutting thereto. Resilient members 22a and 22b extend along body region 37, positioned in between its long edges immediately adjacent to the inside of each long edge.

The preferred materials for the base and cover members may be selected from a range of widely available, preferably medical grade, flexible fabrics or thermoplastic films that are breathable or permeable. The most preferable fabric is a synthetic nonwoven, and the most preferred thermoplastic film is from the class of polyurethane (PU) films from about 20 microns to about 60 microns thick. However, a PE film treated with a plurality of microporous openings may also be used.

An MVTR of at least 200 grams per square meter per 24 hours (GSM/24 hr.), is a most preferred minimum for the base and cover member materials. MVTR may be determined by a standard test such as, for example, ASTM F1249, ASTM E96, or ASTM2622. A layer of pressure sensitive adhesive, preferably biocompatible with external human tissue, may be disposed on a flat surface side of the preferred material and covered by a protective, removable, release liner. The disposition of adhesive may be fully coextensive with the preferred material surface area. To increase MVTR, the disposition may be pattern-coated such that a lesser amount of adhesive covers the entire surface area.

The preferred material for resilient members 22 is a thermoplastic resin that may be selected from a range having flexural, tensile and elastic moduli so as to have substantial in-plane rigidity and out-of-plane flexibility at a thickness preferably greater than 0.010". A most preferred thermoplastic material is a biaxially oriented polyester resin, Poly(ethylene terephthalate) or PET (or boPET). PET is used in a number of medical device applications, is particularly suitable because of its resiliency or spring biasing properties, and is widely available as a medical/industrial commodity.

It should be briefly noted that it may be possible to utilize laser technology to perforate otherwise impermeable thermoplastic resin materials. However, the process may be unsuitable for nasal dilator manufacture on the economic scale required for wide commercial distribution. Furthermore, the perforations could reduce resiliency, requiring that the resilient member have a greater surface area to make up for the deficit.

The vertically stacked layers of dilator 10 may be arranged in several ways. The stacking order of dilator layers, including a peripheral defining layer, may be determined by the preferred material used.

For example, in Figs. 1-3 base member 14 is the peripheral defining layer and is preferably formed from a thermoplastic film, most preferably a PU film. A cover layer is eliminated, so resilient members 22a and 22b are uppermost in the stacking order of layers and are exposed and visible. In Figs. 4-7, for example, cover member 18 is the peripheral defining layer. It may be formed from either thermoplastic film or a nonwoven fabric and is uppermost in the stacking order. Where cover member 18 is the peripheral defining layer, base member 14 is most preferably formed from a nonwoven fabric.

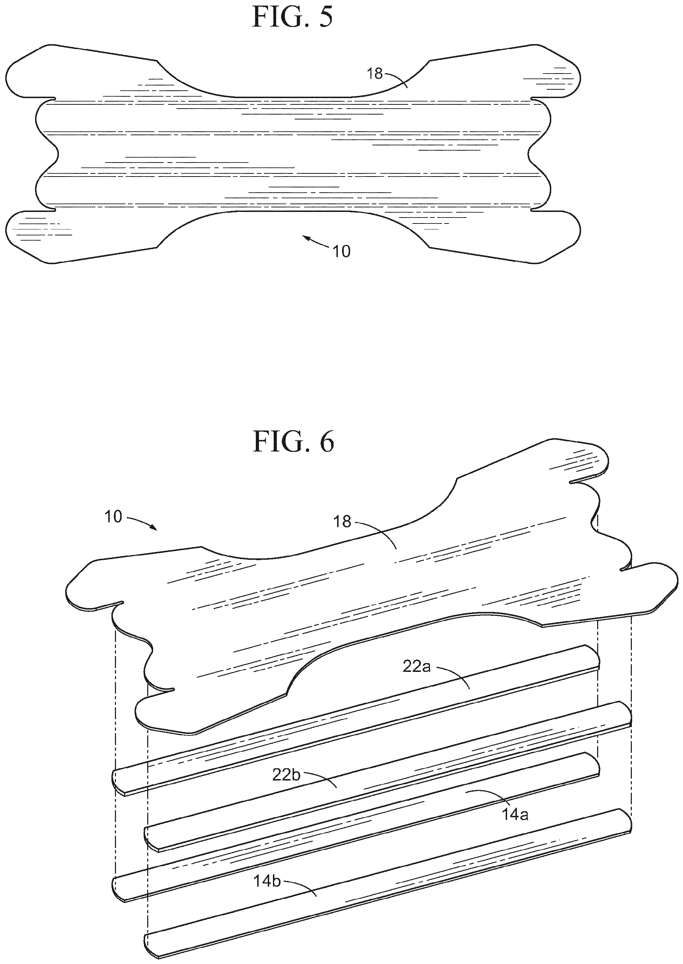

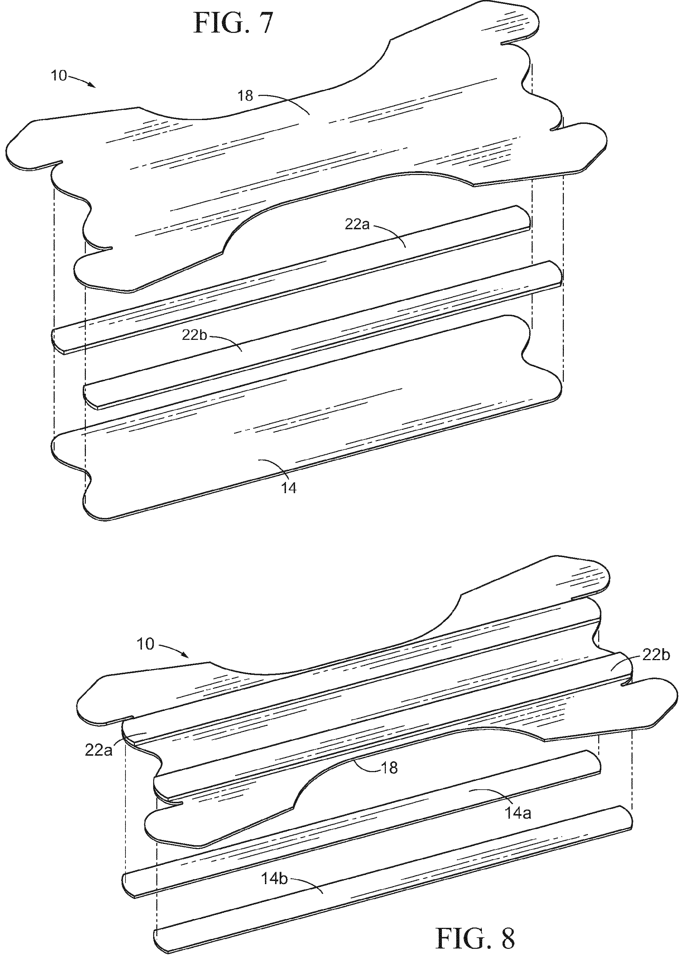

As seen in Fig. 6, the base and resilient layers may have identical plan view peripheries. That is, base members 14a and 14b are coextensive with the surface areas of resilient members 22a and 22b, respectively. Alternatively, base members 14a and 14b may be each slightly wider than either resilient member (not shown). Further alternatively, a single base member 14 may have a periphery encompassing both flat surface areas of resilient members 22, as seen, for example, in Fig. 7.

Where the base layer is made of a flexible fabric and has significantly less surface area than the cover layer, adhesive on the skin-engaging side of the base layer may be optionally eliminated. With or without adhesive, the base layer may also serve as a compressible buffer between the nasal dilator and the skin engaged thereby.

Yet another stacking order is illustrated in Fig. 8. Cover member 18 is preferably formed from a PU film; base members 14a and 14b are secured to the underside of cover member 18, and resilient members 22a and 22b are secured on top of cover member 18. Base members 14a and 14b are aligned with the resilient members, and are intended to absorb moisture vapor from the skin, which will not pass through the impermeable resilient members. Base members 14a and 14b are most preferably formed from a flexible spun-laced nonwoven fabric.

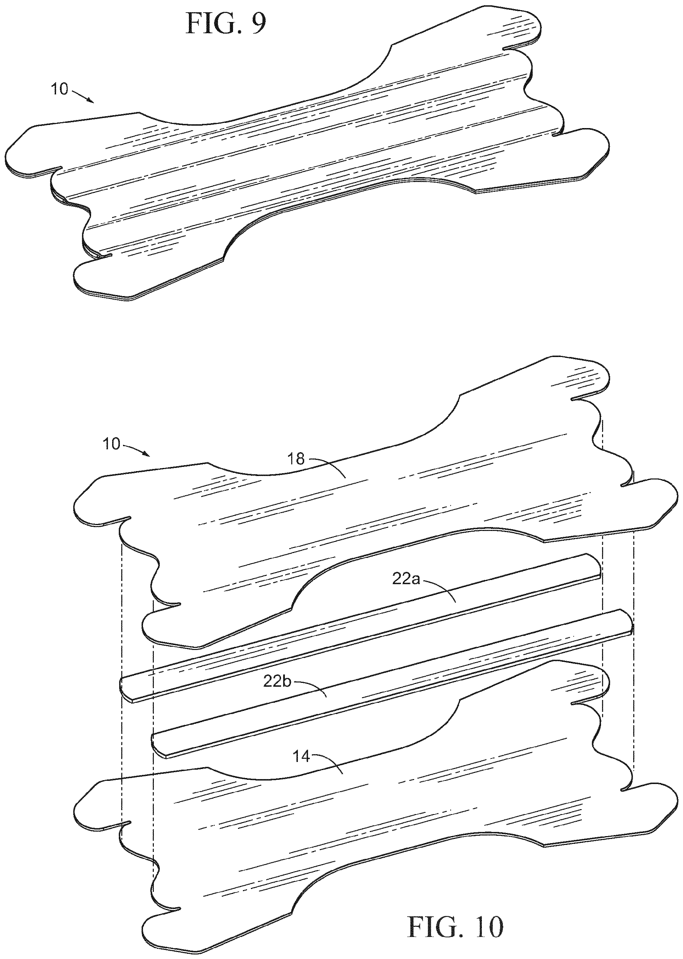

Alternatively, base member 14 and cover member 18 may have substantially similar or identical peripheries, as seen, for example, in Figs. 9-10. In this instance, either base member 14 or cover member 18 may be formed from either thermoplastic film or nonwoven fabric. However, a single peripheral defining layer is most preferred.

Where it is formed from a PU film, the layer so formed most preferably has a sufficient thickness so as to be dimensionally stable without use of a supplemental carrier liner or stabilizing material layer. It may be apparent to one of ordinary skill in the art that unsupported ultra-thin PU film, for example, typically having a thickness of about 15 microns, is generally difficult to handle, prone to curling in on itself, and thus must be supported by a supplemental carrier liner until the article in question is secured to the skin. Accordingly, a single peripheral defining layer formed from ultra-thin PU film may be less preferable.

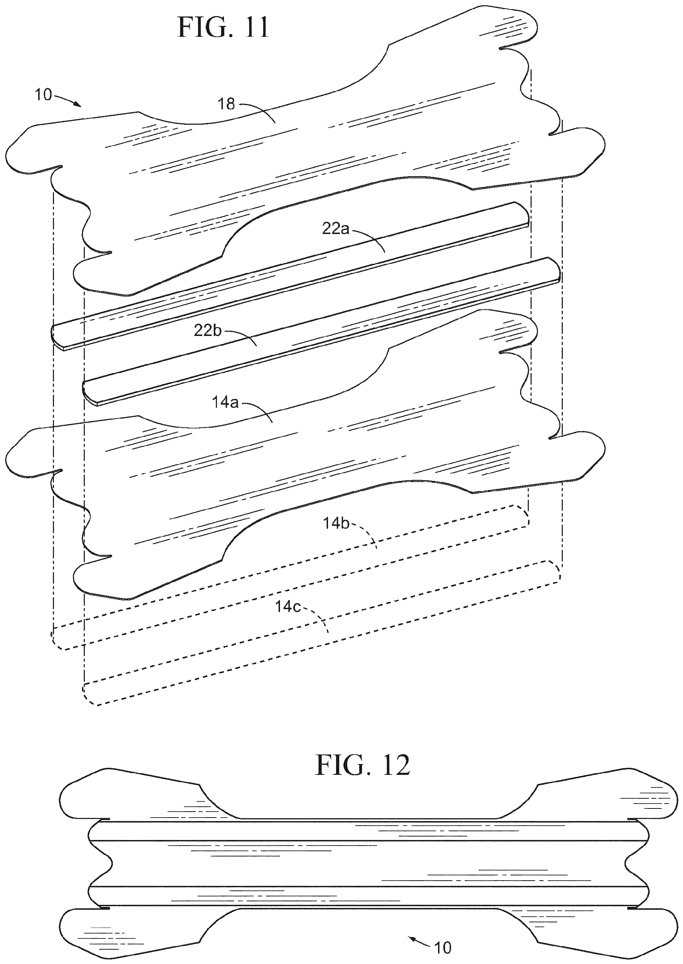

Nonetheless, Fig. 11 illustrates that resilient members 22a and 22b may be sandwiched between cover layer 18 and base layer 14 each formed from thin or ultra-thin PU film (from about 12 to about 19 microns thick for each of the two layers, for example), such that the combined thickness thereof renders dilator 10 dimensionally stable when the protective layer of release liner is removed from dilator 10 preliminary to application to nose 11. Base member 14a preferably has the same or similar periphery as cover member 18, and may have a somewhat lesser surface area. Fig. 11 further illustrates that base members 14b and 14c, depicted in shadow lines, may be optionally placed on the underside of base member 14a, aligned to the surface areas of resilient members 22a and 22b, so as to absorb moisture vapor from the skin as described hereinbefore.

The range of spring biasing force provided by resilient members 22a and 22b combined is most preferably consistent with that of the prior art nasal dilators shown in Figs. 18 and 19, which have three or two resilient member bands, respectively. U.S. Pub. No. 20110000483 teaches that *"In one embodiment, the total spring force delivered by the resilient element as a whole should be from 25 gm to about 35 gm."* U.S. Pat. No. 5533503 teaches that *"The nasal dilator 10, of the present invention, is constructed to produce from 20 to 30 grams of dilating spring biasing force..."*

Accordingly, dilator 10 depicted in Figs. 1-11 most preferably provides spring biasing force in a range of from about 25 grams to about 35 grams, consistent with the 3-band prior art nasal dilator shown in Fig. 18. Dilator 10 depicted in Figs. 12-17 most preferably provides spring biasing force in a range of from about 20 grams to about 30 grams, consistent with the 2-band prior art nasal dilator shown in Fig. 19.

Spring biasing force is determined by resilient member dimensions. For example, one to three resilient members formed from PET, each measuring about 2.0" in length, having a width/combined width of about 0.25", and a thickness of 0.010", generate roughly 25 grams of spring biasing force. Resilient members 22a and 22b each preferably have a width less than about 0.12", and may have a thickness greater than 0.010" so as to arrive at the preferred range of spring biasing force. If necessary, two or more resilient members may be overlaid or stacked one atop another so as to arrive at a combined thickness greater than 0.010". Where resilient members 22 are stacked or overlaid, their length and width are preferably substantially the same.

As noted hereinbefore, nasal dilators of the present invention are drawn to scale. The prior art nasal dilators shown in Figs. 18 and 19, being widely available to the public and so physically measurable, are also drawn to scale. The prior art nasal dilators and nasal dilators of the present invention are drawn to size relative to each other, particularly as seen in those drawing figures in which they are specifically compared, namely Figs. 18-22, 25-26, and 27-28.

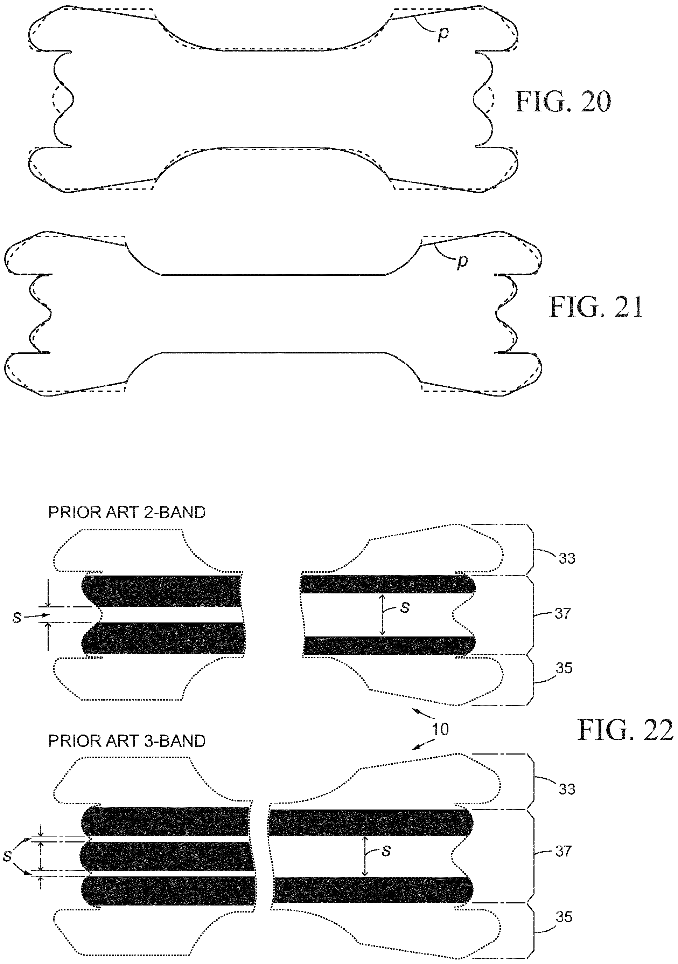

In that regard, Fig. 20 shows a periphery, p, of dilator 10 as shown in Figs. 1-11, overlaid onto the periphery of the prior art 3-band nasal dilator, which is depicted in dashed lines. Similarly, Fig. 21 shows periphery p of dilator 10 as shown in Figs. 12-17, overlaid onto the periphery of the prior art 2-band nasal dilator, also depicted in dashed lines. It should be visually apparent that the overall length and width of the nasal dilators and their peripheries are substantially the same. Furthermore, small differences between the dilator peripheries substantially offset, such that the surface areas of the prior art nasal dilators and nasal dilator 10 are nearly identical.

It may also be visually apparent that the width of body region 37 and corner tab regions 33 and 35 of dilator 10 are substantially the same as corresponding regions of the prior art nasal dilators. In this regard Fig. 22 shows fragmentary plan views of dilator 10 aligned to corresponding fragmentary plan views of the prior art nasal dilators. For all dilators, the width of body region 37 is about 45% of the dilator overall width; corner tab regions 33 and 35, combined, are about 55% of dilator overall width.

Thus far it has been demonstrated that resiliency (spring biasing force), periphery, overall surface area, body region width and corner tab region width are substantially the same, if not nearly identical, between nasal dilators of the present invention and the prior art nasal dilators.

Fig. 22 shows that resilient members 22 of dilator 10 occupy significantly less surface area than resilient members of the prior art nasal dilators, which is discussed further hereinbelow. (Resilient members are depicted in solid black for illustrative emphasis.) Additionally, a lateral distance, *s*, extends between the inside long edges of the resilient members. Distance s is about 2.7 times greater in dilator 10 compared to the 2-band prior art nasal dilator, and about 3.6 times greater than the prior art 3-band nasal dilator. The relative narrow width of the resilient members and the spacing between them denoted by distance s creates a substantial surface area within body region 37 that may be permeable rather than impermeable.

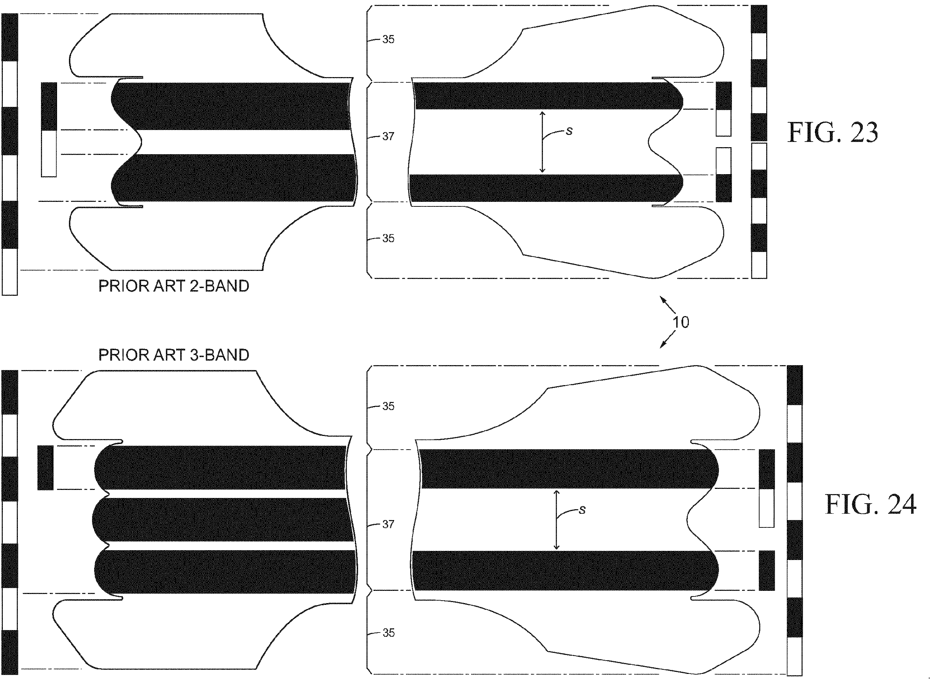

Since resilient members of both the prior art nasal dilators and dilator 10 extend fully from end to end, width comparisons may be viewed as one measure of the difference between impermeable (resilient member) surface area and permeable (engagement element) surface area, the latter being greater in dilators of the present invention compared to the prior art nasal dilators. To further compare widths, Figs. 23 and 24 show a fixed unit of measure represented by black and white blocks. Each block unit is equal to the width of one resilient member of the nasal dilator adjacent thereto.

Fig. 23 shows distance s of dilator 10 being greater than two block units. For the prior art 2-band nasal dilator, space between the two resilient members is less than one-half unit. For dilator 10, one resilient member width is equal to about one-tenth (9.9%) of the overall width of dilator 10; both resilient members combined are about one-fifth (19.8%) thereof. The corresponding prior art 2-band nasal dilator resilient member width is greater, about two-elevenths (18.3%) of overall dilator width; both resilient members combined are slightly more than two-fifths (36.6%) thereof. The prior art nasal dilator resilient members' combined width occupies 1.85 times more of the dilator's overall width than do resilient members of the present invention. (Conversely, the combined width of resilient members of the present invention occupy 1.85 times less of the dilator's overall width than the resilient members of the prior art nasal dilator.)

Similarly, in Fig. 24, distance s of dilator 10 is about one and one-half block units. For the prior art 3-band nasal dilator, combined space between the resilient members is less than one-half unit. For dilator 10, one resilient member width is equal to about one-eighth (12.6%) of the overall width of dilator 10; both resilient members combined are about one-fourth (25.1%) thereof. The corresponding prior art 3-band nasal dilator resilient member width is about one-seventh (14.5%) of overall dilator width; both resilient members combined are slightly less than three-sevenths (43.6%) thereof. The individual resilient member widths for both dilators are similar, but dilator 10 uses two resilient members instead of three. So, the three prior art resilient members' combined width occupies about 1.74 times more of the dilator's overall width. (Conversely, the combined width of two resilient members of dilator 10 occupy 1.74 times less of the dilator's overall width than do three resilient members of the prior art nasal dilator.)

Comparing body region width to combined resilient member width, Fig. 23 shows that resilient members 22a and 22b occupy about 45.2% of the body region width. Thus, body region width is about 2.2 times greater than the combined resilient member width. However, for the adjacent prior art 2-band nasal dilator, body region width is just 1.26 times the combined width of both resilient members; both resilient members combined are about 79.4% of body region width. The prior art resilient members thus occupy about 1.75 times more of the body region width than do resilient members of corresponding dilator 10 adjacent thereto.

Similarly, Fig. 24 shows that resilient members 22a and 22b occupy about 55.2% of body region width. Thus, body region width is about 1.8 times greater than the combined resilient member width. However, for the adjacent prior art 3-band nasal dilator, body region width is just 1.13 times the combined width of three resilient members; the three resilient members combined are about 88.2% of body region width. The prior art resilient members combined thus occupy about 1.6 times more of body region width than do the two resilient members of corresponding dilator 10 adjacent thereto.

**Table 1.0 summarizes the aforementioned width comparisons:**

| Table 1.0 | dilator 10 Fig. 23 | prior art 2-band, Fig. 23 | dilator 10 Fig. 24 | prior art 3-band, Fig. 24 |
|---|---|---|---|---|
| distance s | > 2 units | < 1/2 unit | ∼1.5 units | < 1/2 unit |
| one resilient member width to dilator width | 1/10th (9.9%) | 2/11th (18.3%) | 1/8th (12.6%) | 1/7th (14.5%) |
| combined resilient member widths to dilator width | 1/5th (19.8%) 1.85 x less | 4/11th (36.6%) 1.85 x more | 1/4th (25.1%) 1.74 x less | 3/7th (43.6%) 1.74 x more |
| body region width > combined resilient member widths | 2.2 x greater | 1.26 x greater | 1.8 x greater | 1.13 x greater |
| combined resilient member widths as a % of body region width | 45.2% 1.76 x less | 79.4% 1.76 x more | 55.2% 1.6 x less | 88.2% 1.6 x more |

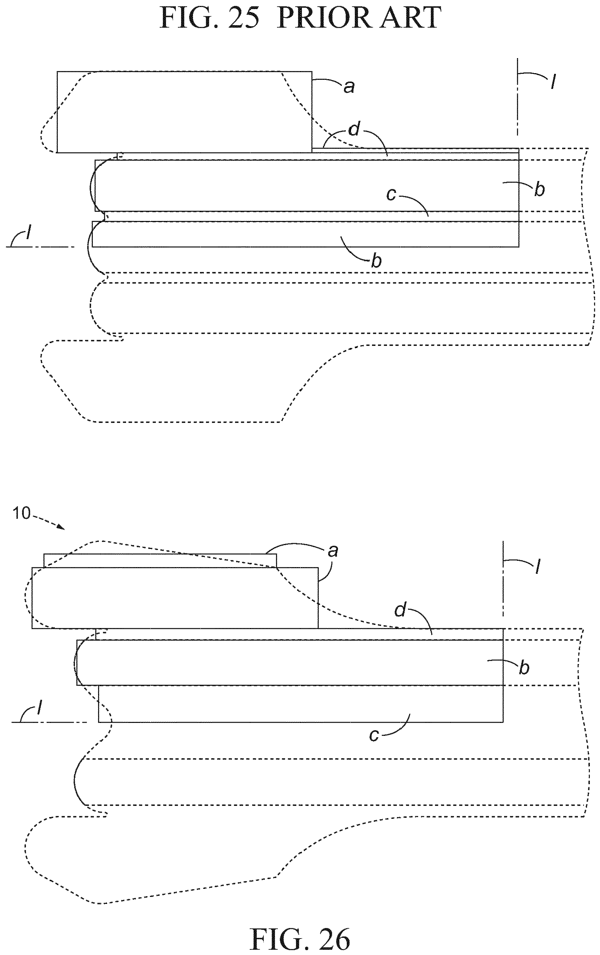

Further comparison of differences in impermeable resilient member surface area and permeable engagement element surface area is shown in Figs. 25-28. To help visualize surface area comparison, various-sized rectangles are overlaid onto one quadrant of each nasal dilator whereby to measure particular surface areas: corner tab, ***a***; resilient member, ***b*;** space between resilient members, ***c***; and a narrow space, ***d***, between corner tab ***a*** and resilient member ***b***. Since the nasal dilators are symmetric about their longitudinal and lateral centerlines, ***l***, and all four quadrants are mirror images, only one quadrant is measured. According to these measurements, the total plan view surface area of dilator 10 (as seen in Figs. 1-11) is about 2.7% less than the 3-band prior art nasal dilator (seen in Fig. 18), while the total plan view surface area of the 2-band prior art nasal dilator is about 0.5% less than dilator 10 (as shown in Figs. 12-17).

Surface areas ***a***, ***c*** and ***d*** are part of the engagement element extending in between and outward from resilient member surface areas ***b***. As discussed hereinbefore, surface areas ***a***, **c** and ***d*** are permeable, allowing moisture vapor to pass from the skin therethrough (i.e., MVTR); surface areas ***b*** do not allow moisture vapor transmission, regardless of the permeability of the engagement material to which the resilient member is secured. Note that surface areas ***a*** and ***d*** correspond to corner tab regions, which, combined, average about 40.1% of the total plan view surface area for both the prior art nasal dilators and dilator 10. Surface area c corresponds to the body region, which averages 59.1% thereof.

Overall, dilator 10 has roughly 1.4 times more permeable engagement element surface area, and from about 1.6 to 1.7 times less impermeable surface area, compared to the prior art nasal dilators. However, nearly all of the greater permeable surface area is within body region 37. There dilator 10 has from about 2.8 to about 3.6 times more permeable surface area. The ratio of permeable to impermeable surface area for the prior art nasal dilators is roughly 1:1, while for dilator 10 it's from about 2.1:1 to about 2.5:1, which is about 2.3 to 2.4 times greater. Specific detail follows, summarized in Tables 2.0-2.3.

Beginning with the prior art 3-band nasal dilator, Fig. 25 shows engagement element surface area ***c*** being about 7% of total plan view surface area. Surface areas ***a*** and ***d*** combined are about 40.1% thereof. Added together, surface areas ***a***, ***c*** and ***d*** are about 47% of the total plan view surface area. Resilient member surface area ***b*** is about 53% of the total plan view surface area. So, the ratio of permeable engagement element surface area to non-permeable resilient member surface area is not quite equal, at about 0.9:1 (47.1 / 52.9 = 0.890 to be more exact).

Fig. 26 shows corresponding dilator 10 (as illustrated in Figs. 1-11). Engagement element surface area ***c*** is almost 3.6 times greater than the prior art 3-band nasal dilator, and is about 25% of total plan view surface area. Surface areas ***a*** and ***d*** combined are about 42.4% of the total plan view surface area. Added together, areas ***a***, ***c*** and ***d*** are about 67.4% of total plan view surface area. Resilient member surface area ***b*** is about 32.6% of the total plan view surface area, which is 1.62 times less than the prior art 3-band nasal dilator. The ratio of permeable engagement element surface area to non-permeable resilient member surface area is just over 2:1 (67.4 / 32.6 = 2.068 to be more exact). That ratio is more than double -about 2.3 times greater- than the 0.9:1 ratio of the prior art 3-band dilator.

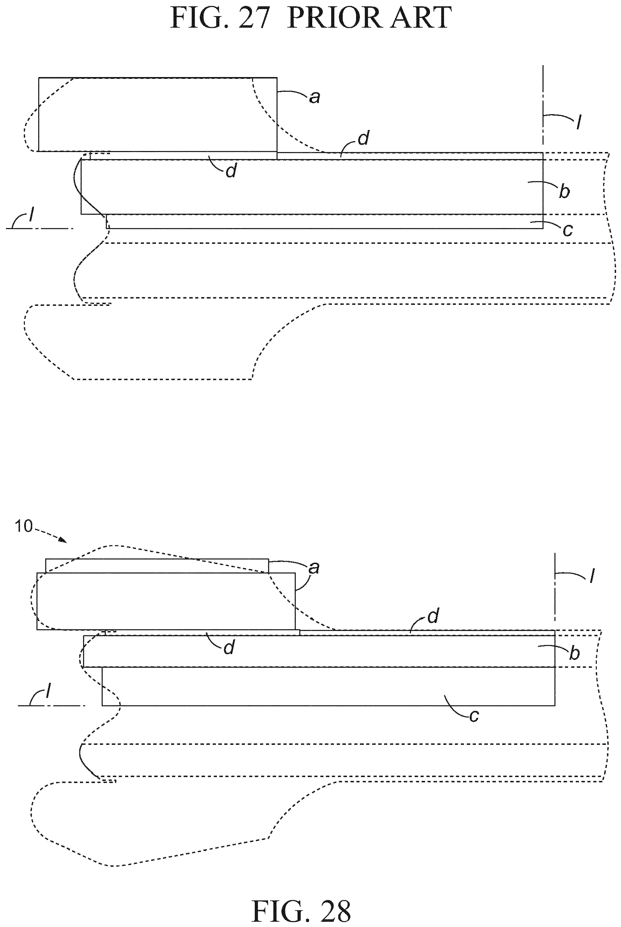

Turning now to the prior art 2-band nasal dilator, Fig. 27 shows engagement element surface area ***c*** being about 12% of total plan view surface area. Surface areas ***a*** and ***d*** combined are about 39.4% thereof. Added together, surface areas ***a***, ***c*** and ***d*** are about 51.4% of total plan view surface area. Resilient member surface area ***b*** is about 48.6% of the total plan view surface area. So, the ratio of permeable engagement element surface area to non-permeable resilient member surface area is about equal at 1:1 (51.4 / 48.6 = 1.06 to be more exact).

Fig. 28 shows corresponding dilator 10 (as seen in Figs. 12-17). Engagement element surface area ***c*** is about 2.8 times greater than the corresponding prior art 2-band nasal dilator, and is about 33% of total plan view surface area. Surface areas ***a*** and ***d*** combined are about 38.5% thereof. Added together, surface areas ***a***, ***c*** and ***d*** are about 71.6% of total plan view surface area. Resilient member surface area ***b*** is about 28.4% of the total plan view surface area, which is about 1.7 times less than the corresponding prior art 2-band nasal dilator. So, the ratio of permeable engagement element surface area to non-permeable resilient member surface area is about 2.5:1 (71.6 / 28.4 = 2.52 to be more exact). That ratio is nearly 2.4 times greater than the 1:1 ratio of the prior art 2-band dilator.

**Table 2.0 summarizes the aforementioned a-d surface areas. Additionally, resilient member surface area b and engagement element surface area c combined reflect the total surface area for body region 37 (shown in the last column). Engagement element surface areas a and d combined reflect the surface area for corner tab region 33 or 35 (shown in the first column). Adding the first and last columns together equals 100% of the total nasal dilator plan view surface area. Extrapolating from Table 2.0 shows that permeable surface area makes up from about 43% to 54% of body region 37 (25.0 / 57.6 = 43.4 and 33.1 / 61.5 = 53.8). Further extrapolating from Table 2.0 shows that resilient member surface area b occupies from about 46.2% to about 56.6% of the body region surface area (28.4 / 61.5 = 46.2 and 32.6 / 57.6 = 56.6).**

| **Table 2.0** % of total plan view surface area | engagement element surface area (*a* + *d*) | engagement element surface *area* (*a* + *c* + *d*) | engagement element surface area (*c*) | resilient member surface area (*b*) | body region surface area |
|---|---|---|---|---|---|
| prior art 3-band | 40.1% | 47.1% | 7.0% | 52.9% | 59.9% |
| dilator 10 Figs. 1-11 | 42.4% | **67.4%** (1.43 x more) | **25.0%** (3.57 x more) | **32.6%** (1.62 x less) | 57.6% |
| prior art 2-band | 39.4% | 51.4% | 12.0% | 48.6% | 60.6% |
| dilator 10 Figs. 12-17 | 38.5% | **71.6%** (1.39 x more) | **33.1%** (2.76 x more) | **28.4%** (1.71 x less) | 61.5% |
| *average for all dilators* | *40.1%* | | | | *59.9%* |

**Table 2.1 summarizes the ratios between combined engagement element surface areas a, c and d and resilient member surface areas b.**

| **Table 2.1** % of total plan view surface area | engagement element surface area (*a* + *c* + *d*) | resilient member surface area (*b*) | ratio (*a* + *c* + *d*) to (*b*) | times greater |
|---|---|---|---|---|
| prior art 3-band | 47.1% | 52.9% | 0.887:1 | |
| dilator 10 Figs. 1-11 | 67.4% | 32.6% | **2.068:1** | 2.33 |
| prior art 2-band | 51.4% | 48.6% | 1.058:1 | |
| dilator 10 Figs. 12-17 | 71.6% | 28.4% | **2.521:1** | 2.38 |

**Table 2.2 summarizes the above-described greater permeable engagement element surface area of dilator compared to the prior art nasal dilators:**

| **Table 2.2** % of total plan view surface area | engagement element surface area (*a* + c + *d*) | surface area times greater | engagemen t element surface area (c) | surface area times greater | distances (width) times greater |
|---|---|---|---|---|---|
| prior art 3-band | 47.1% | | 7.0% | | |
| dilator 10 Figs. 1-11 | **67.4%** | 1.43 | **25.0%** | 3.57 | 3.6 |
| prior art 2-band | 51.4% | | 12.0% | | |
| dilator 10 Figs. 12- 17 | **71.6%** | 1.39 | **33.1%** | 2.76 | 2.7 |

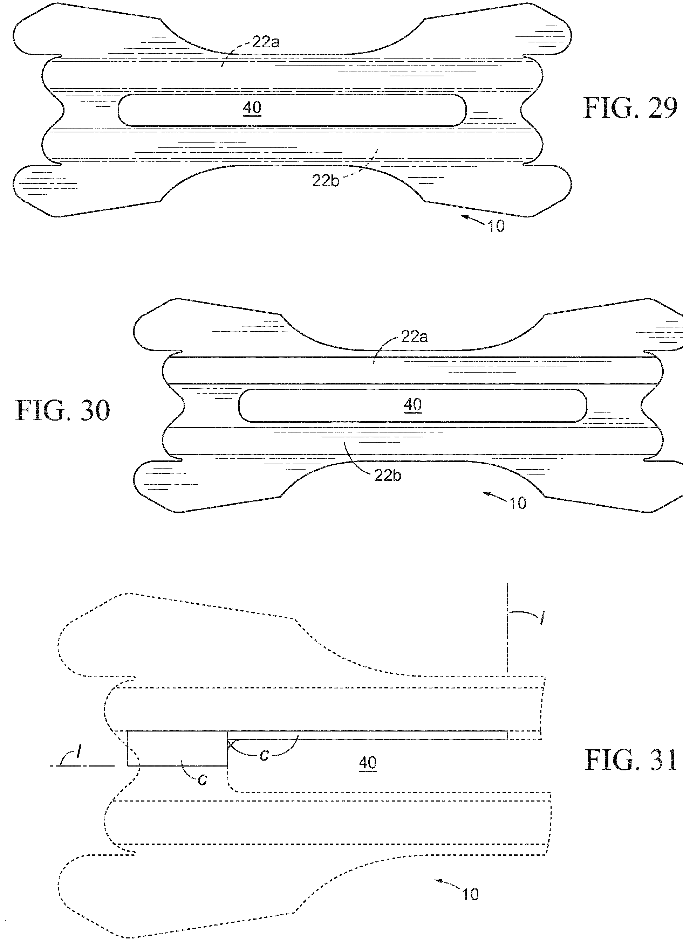

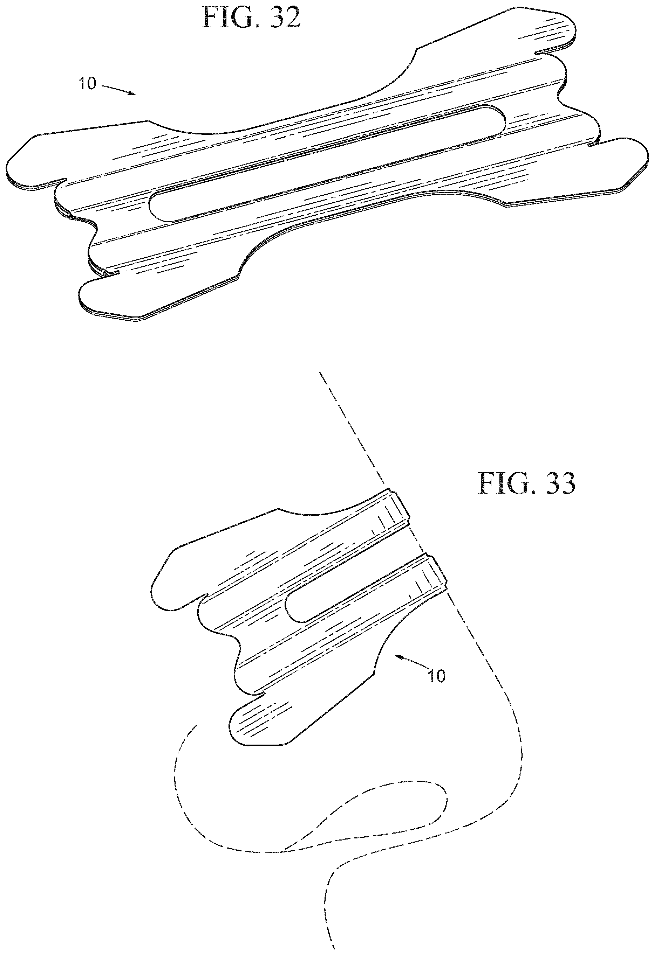

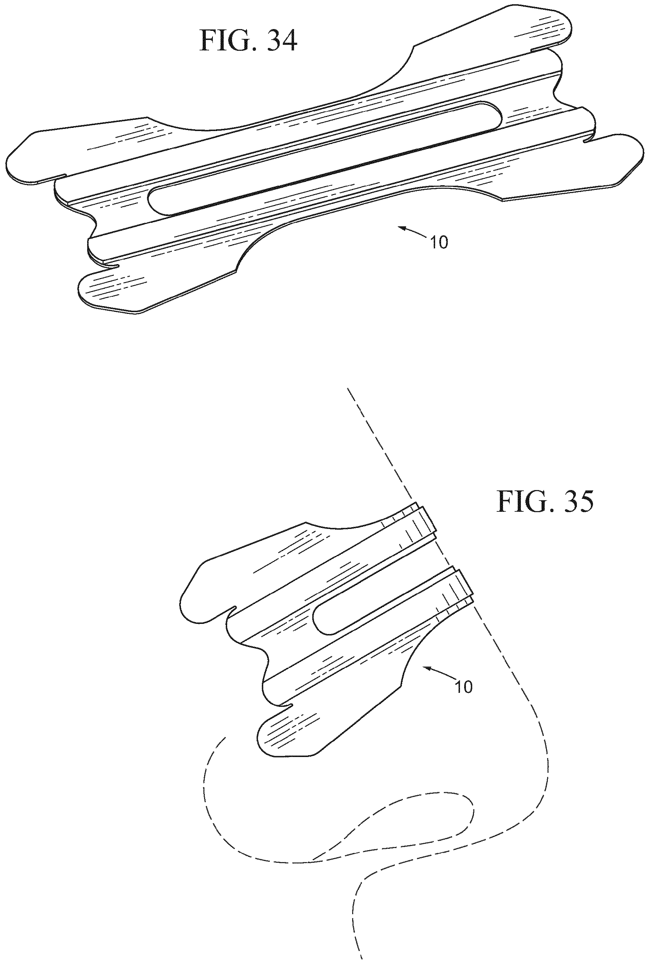

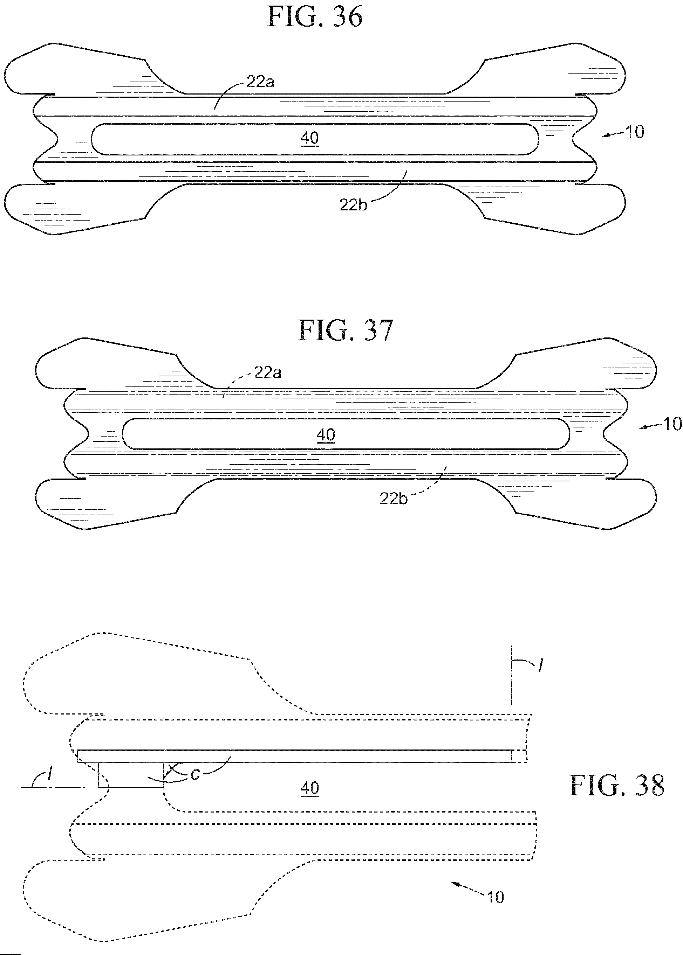

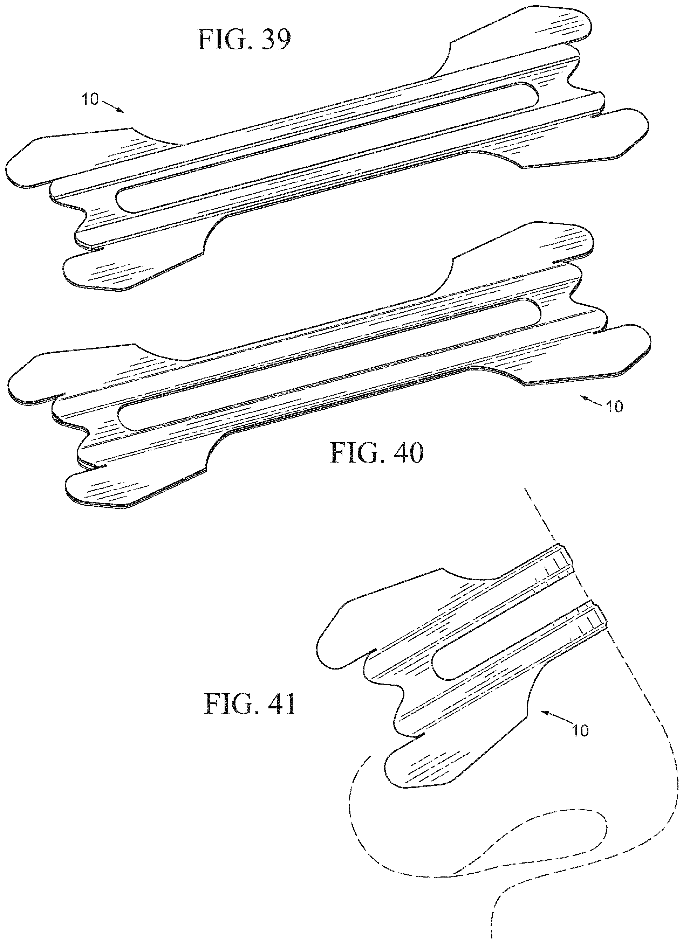

To further increase breathability, Figs. 29-42 illustrate that dilator 10 may include an opening, 40, within engagement element surface area c. As particularly seen in Figs. 31 and 38, opening 40 may displace roughly one-half of surface area c, as seen in Table 2.3.

| **Table 2.3** % of total plan view surface area | opening 40 | surface areas (*a* + *c* + *d*) | resilient member (impermeable) surface areas (*b*) |
|---|---|---|---|
| prior art 3-band | n/a | 47.0% | 52.9% |
| dilator 10 Fig. 25 | 12.2% | 55.2% | 32.6% |
| prior art 2-band | n/a | 51.4% | 48.6% |
| dilator 10 Fig. 28 | 15.2% | 56.4% | 28.4% |

As a result of opening 40, impermeable resilient member surface areas ***b*** remain the same as before for both dilator 10 and the prior art nasal dilators. The combined engagement element surface areas ***a***, ***c*** and ***d*** remain the same as before for the prior art nasal dilators (because they do not have an opening). However, where surface area c was previously 25.0% of the plan view surface area of dilator 10, Fig. 31 shows that opening 40 comprises 12.2% thereof, with the remainder of area c being 12.8% (12.8% + 12.2% = 25.0%). Similarly, Fig. 38 shows that opening 40 comprises 15.2% with the remainder of area c being 17.9% (17.9% + 15.2% = 33.1%).

In view of the increased breathable surface area of dilator 10, a hypothetical MVTR, both with and without opening 40, may be estimated and compared between the prior art nasal dilators and dilator 10 by assigning MVTR values to the previously discussed nasal dilator surface areas.

An MVTR value for the impermeable resilient member surface areas b is obviously zero. MVTR for engagement element surface areas ***a***, ***c*** and ***d*** is based on a rate of 200 grams per square meter over 24 hours (GSM/24 hr.). The MVTR of human skin is believed to be ∼ 200-400 GSM/24 hr. ("What is MVTR and how is it measured?", Avery Dennison Specialty Tape Division, U.S. Avery Dennison Corporation, 2018, stus.averydennison.com/std/ stus.nsf/ed/). For purposes herein, MVTR for opening 40 is based on a rate of 400 GSM/24 hr.

A total plan view surface area of 11.5 square centimeters is used for the prior art 3-band nasal dilator, and 9.9 square centimeters for dilator 10 that corresponds to the prior art 2-band nasal dilator. These surface areas approximate nasal dilator actual size. As discussed hereinbefore, the prior art 2-band nasal dilator surface area was calculated to be about 0.5% less than corresponding dilator 10, and the surface area of dilator 10 corresponding to the prior art 3-band nasal dilator was calculated to be about 2.7% lesser.

Accordingly, surface area differences are reflected in the Total Surface Area column of Table 3.0. MVTR rates are represented as grams per square centimeter (Gcm^2/24hr.), which translates to 0.02 for engagement surface areas and 0.04 for opening 40. At 0.15 Gcm^2/24hr. dilator 10 has more than one-third greater MVTR (36.4%) than the prior art 3-band nasal dilator. At 0.14 Gcm^2/24hr. dilator 10 has about 40% greater MVTR than the prior art 2-band nasal dilator. Where dilator 10 includes opening 40, MVTR is roughly two-thirds greater; at 0.18 and 0.17 Gcm^2/24hr., MVTR is 63.6% and 70% greater, respectively.

Total surface areas, engagement surface area and MVTR values are summarized here:

| **Table 3.0** | total surface area *a*+*b*+*c*+*d* (cm^2) | engagement surface area *a* + *c* + *d* (cm^2) | MVTR Gcm^2/24hr (*a* + *c* + *d*) | greater MVTR as % | increase in MVTR w/opening 40 | greater MVTR as % |
|---|---|---|---|---|---|---|
| prior art 3-band | 11.5 | 5.41 | 0.11 | n/a | | |
| dilator 10 | 11.2 (2.7% less) | 7.55 | **0.15** | 36.4% | **0.18** | 63.6% |
| prior art 2-band | 9.85 (0.5% less) | 4.83 | 0.10 | n/a | | |
| dilator 10 | 9.9 | 7.08 | **0.14** | 40.0% | **0.17** | 70.0% |

It may be apparent to those of ordinary skill in the art that breathable materials used in medical devices may have an MVTR that is greater than 200 GSM/24 hr. However, the preferred PU film material for nasal dilators of the present invention has a thickness so as to be dimensionally stable, as described hereinbefore. In view of that preferred thickness it is believed that 200 GSM/24 hr. is a more realistic value. Similarly, fabric-based dilators typically utilize two nonwoven layers, each with an adhesive coating on one side. It is thus believed that 200 GSM/24 hr. is a reasonable estimated value for nonwoven dilator construction.

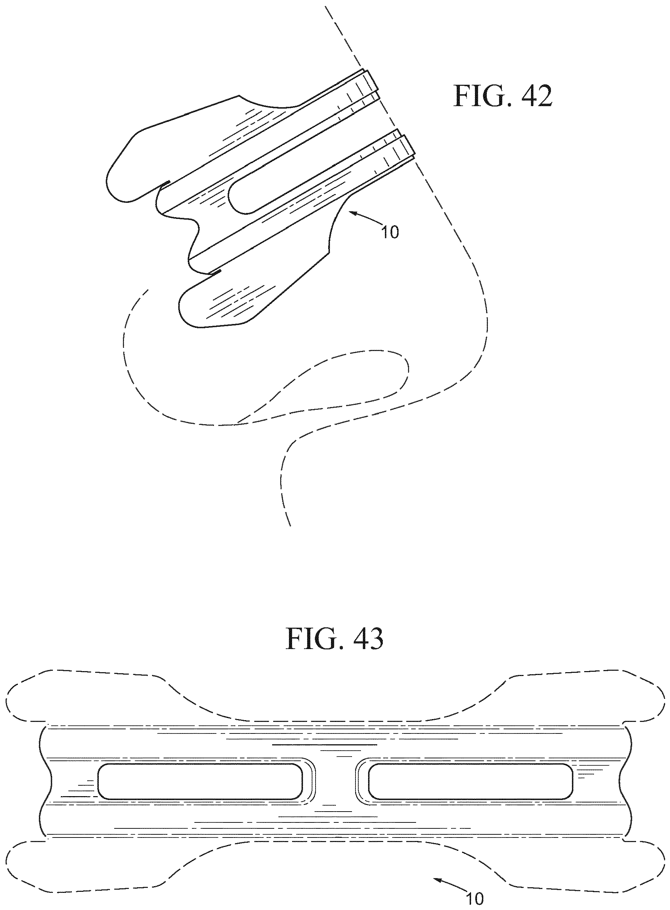

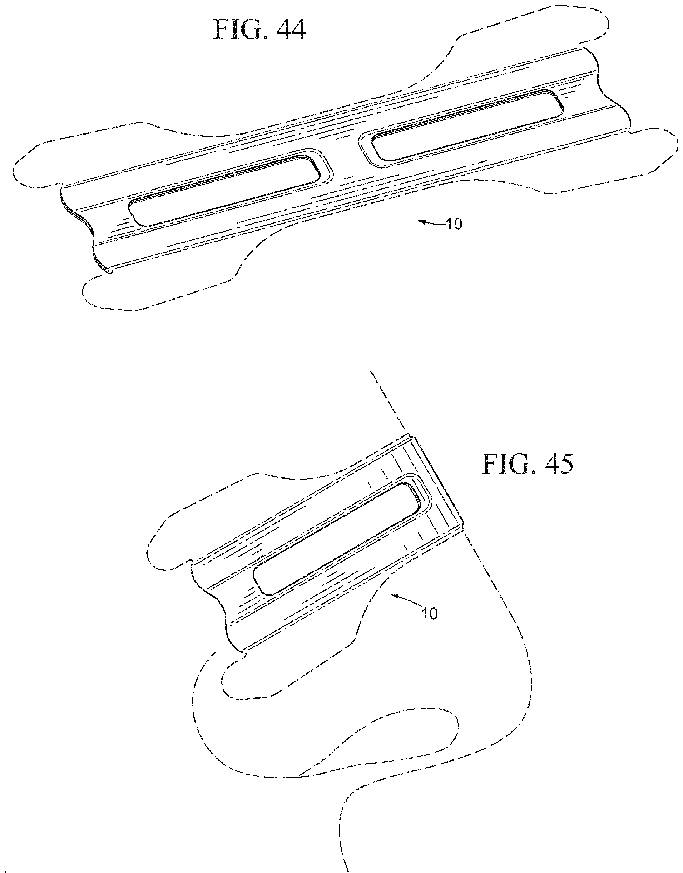

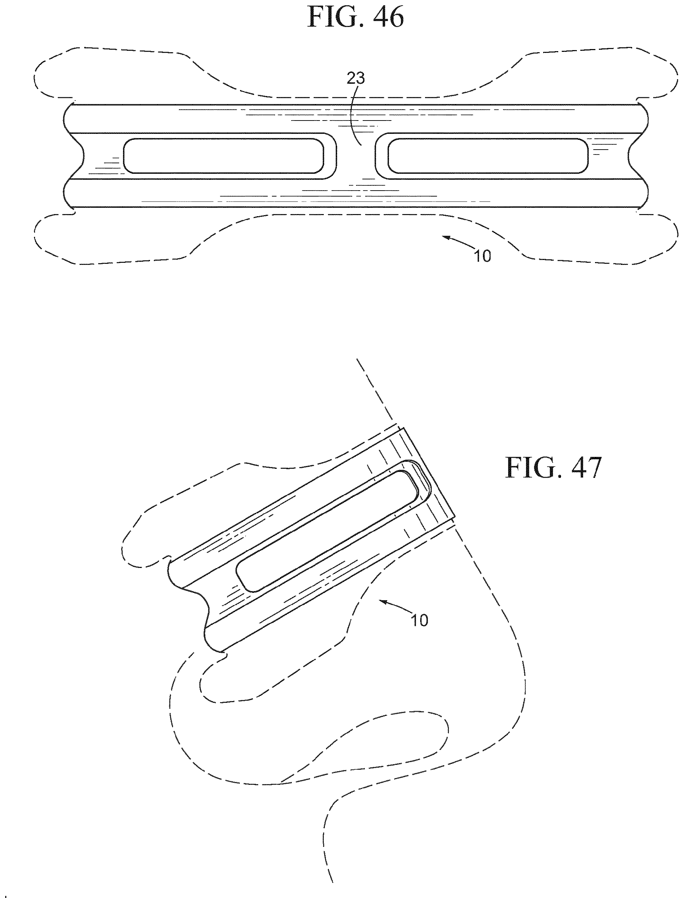

Figs. 43-48 show that resilient members 22a and 22b may be joined together to form a single resilient member 22, preferably at mid-sections thereof, by a web, 23. Web 23 is preferably as narrow as practicable so as to displace as little of engagement element surface area c as possible. Opening 40 may be placed to either or both sides of web 23.

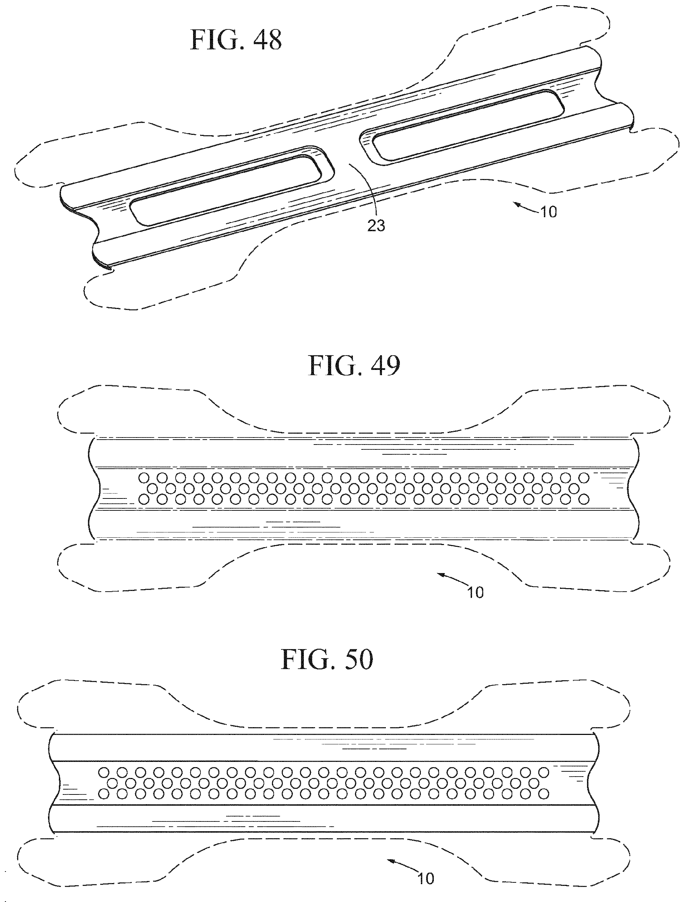

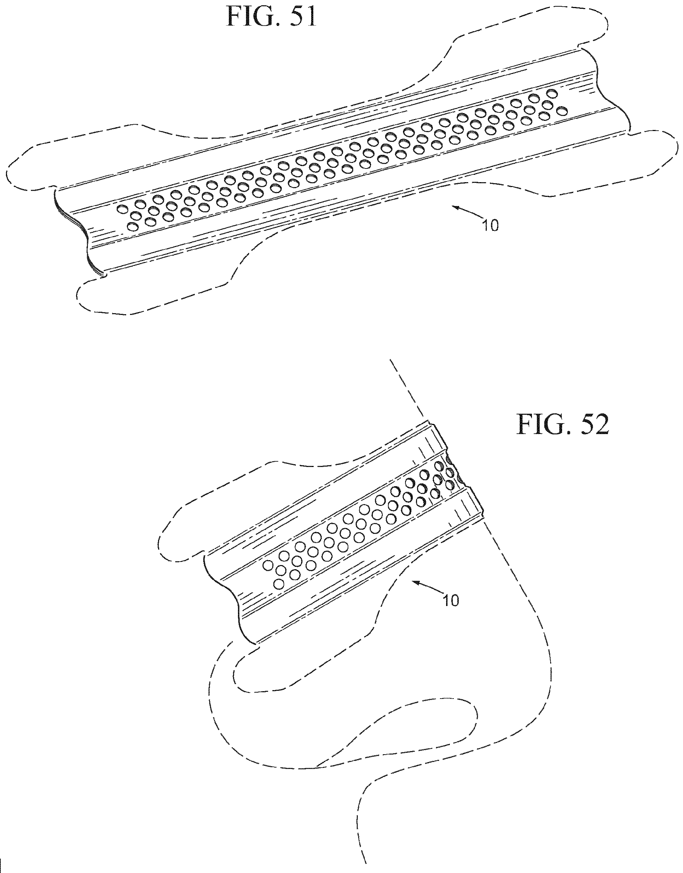

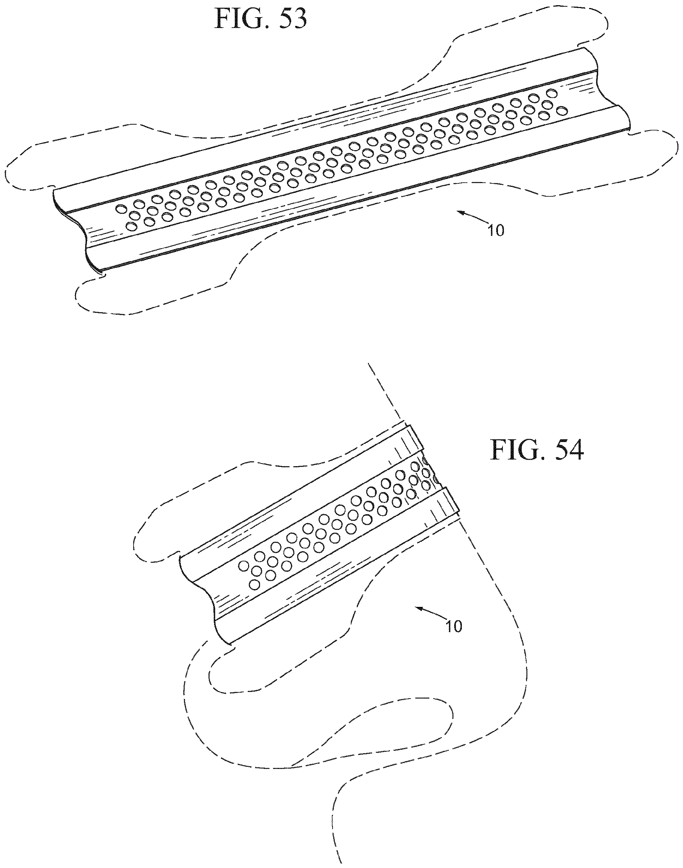

Figs. 49-54 show that a plurality of small openings may be used in lieu of a one or two openings 40, or in lieu of microporous openings (perforations in thin supple films, such as PE film, as discussed hereinbefore). The size, shape and number of the smaller openings may vary from that shown in the drawing figures.

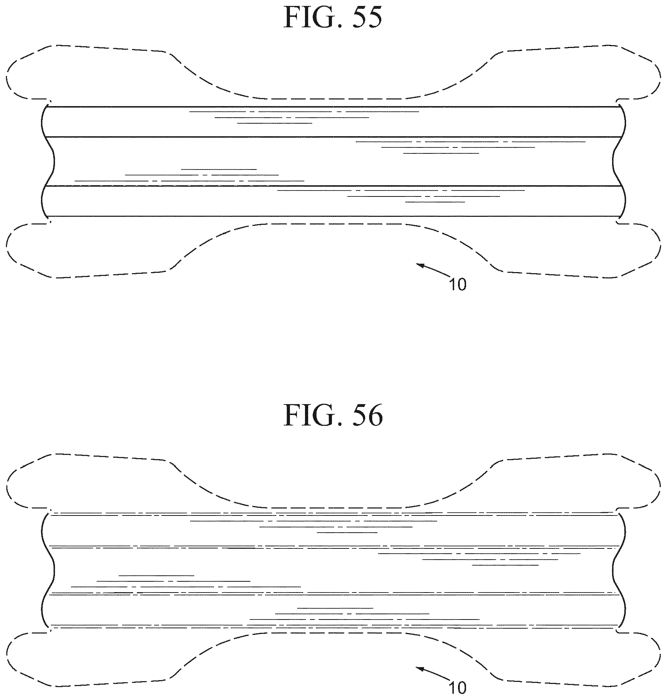

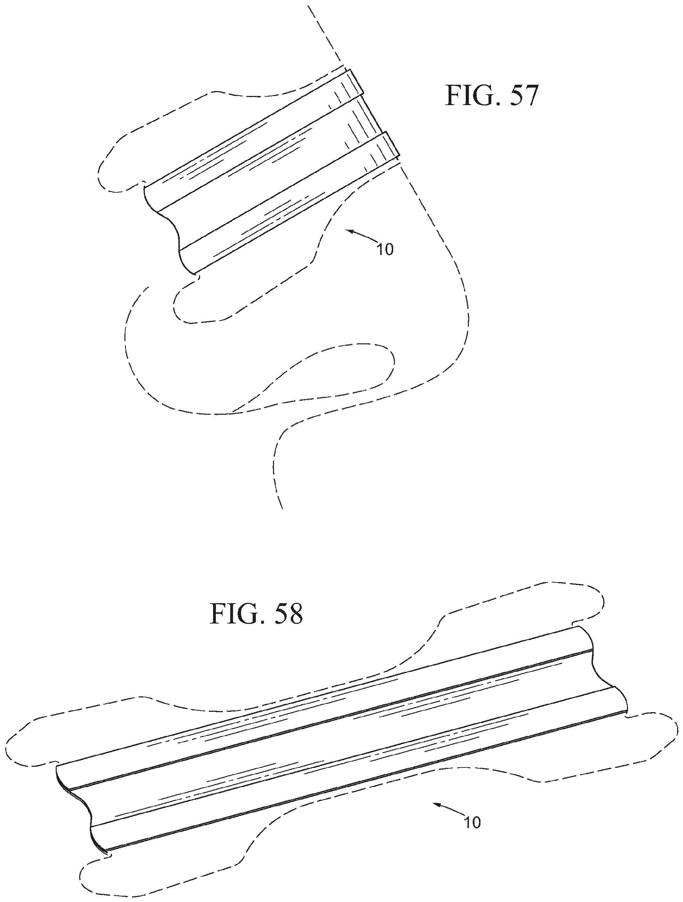

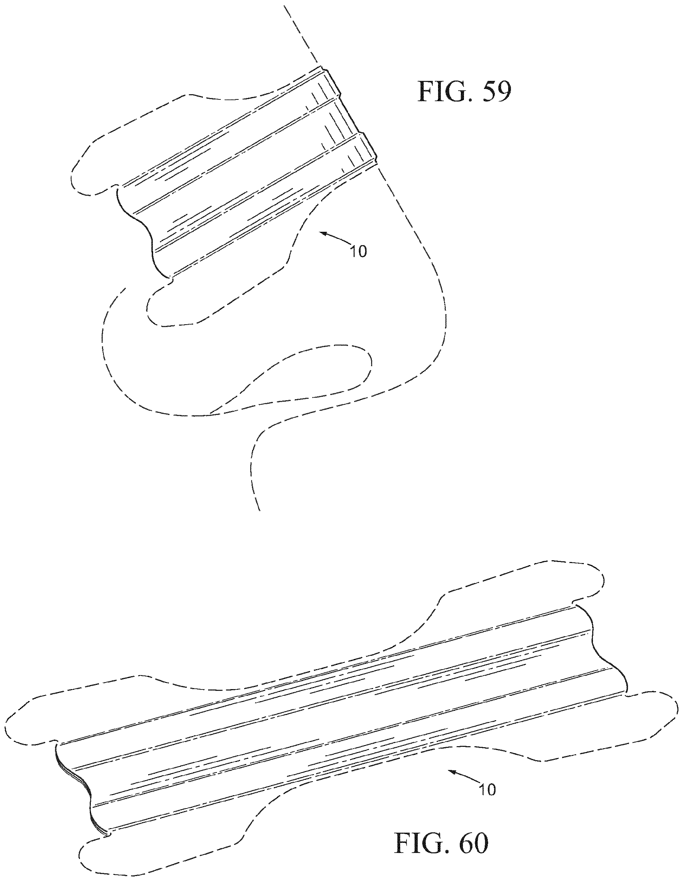

Figs. 55-60 show an embodiment in accordance with the present invention.

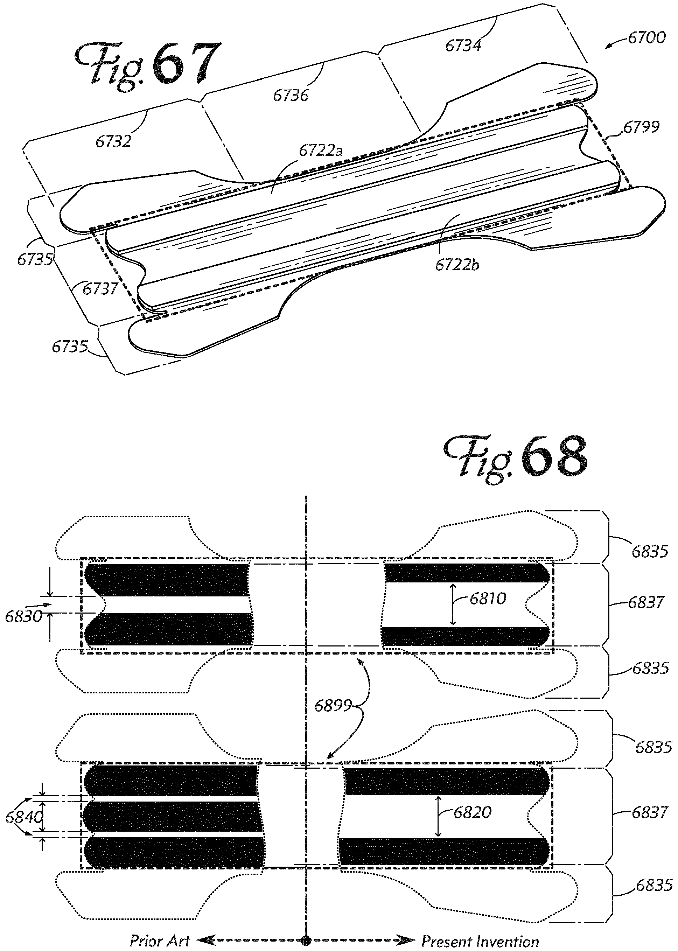

An embodiment of the invention is a dog-bone style nasal dilator, generally similar to the device 6700 depicted in Figure 67. Various regions of the dilator are identified in this figure, and the same terms will be used to refer to corresponding regions in both inventive and prior-art dilators.

Dilator 6700 is an oblong structure which may be divided into three regions along its length: lateral end regions 6732, 6734 are interconnected by a narrower "waist" region 6736. Similarly, across its width, the dilator may be divided into two outer portions 6735 flanking a central band 6737. The central band 6737 includes two resilient members 6722a, 6722b, which extend fully from end to end of the dilator. (End-to-end resilient members allow the dilator to be manufactured more accurately and efficiently, compared to island-placed resilient members.) In use, the narrow waist region 6736 is placed across the bridge of the nose, and the lateral end regions 6732, 6734 are pressed down and adhered against the sides of the nose and the cheeks. The resilient members are thus flexed over the bridge of the nose, and in attempting to spring back to their unflexed configuration, they lift and stabilize the skin to which they are adhered, thus opening and dilating the user's nasal passages. The outer portions 6735 include corner tabs (sometimes called "tab extensions" or "wings") that aid in maintaining the lateral end regions 6732 and 6734 secured to the skin of the nose. The distinguishing characteristics of an embodiment lie mostly within the central band 6737, which may be viewed as a "resilient structure bounding box" 6799, outlined in heavy dashed lines.

Figure 68 compares prior-art dogbone-style two- and three-band nasal dilators with nearly identically-sized two-band dilators according to embodiments of the invention. In this Figure, the resilient members are shown as solid black bars to highlight the substantial portion of the resilient structure bounding box 6899 covered by prior-art resilient members, as compared to the significantly reduced proportion covered by the resilient members in embodiments of the invention. In the prior-art dilators, the resilient members are wider than those of the embodiment, and they are also positioned much closer together (compare 6810, 6820 with 6830, 6840). The result is that the ratio of the total area of the resilient structure bounding box 6899 to the area of the bounding box covered by resilient-member material is much higher in the prior art. Prior-art structures may be as much as 90% covered, whereas an embodiment is 54% or less.

The difference in surface area coverage is important because the material used for all nasal dilator resilient members is typically a nonpermeable or low-permeability plastic such as polyethyl tetraphthalate ("PET" or biaxially-oriented polyethyl tetraphthalate, "boPET"). The other portions of the resilient member bounding box may be covered by a permeable material such as a woven or nonwoven fabric or a breathable plastic film (or, in some embodiments described below, by nothing at all - i.e., by an opening that exposes the skin directly to the atmosphere). By reducing the amount of skin covered by the resilient-member material, an embodiment improves skin breathability and reduces moisture vapor accumulation, and thus improves device comfort.

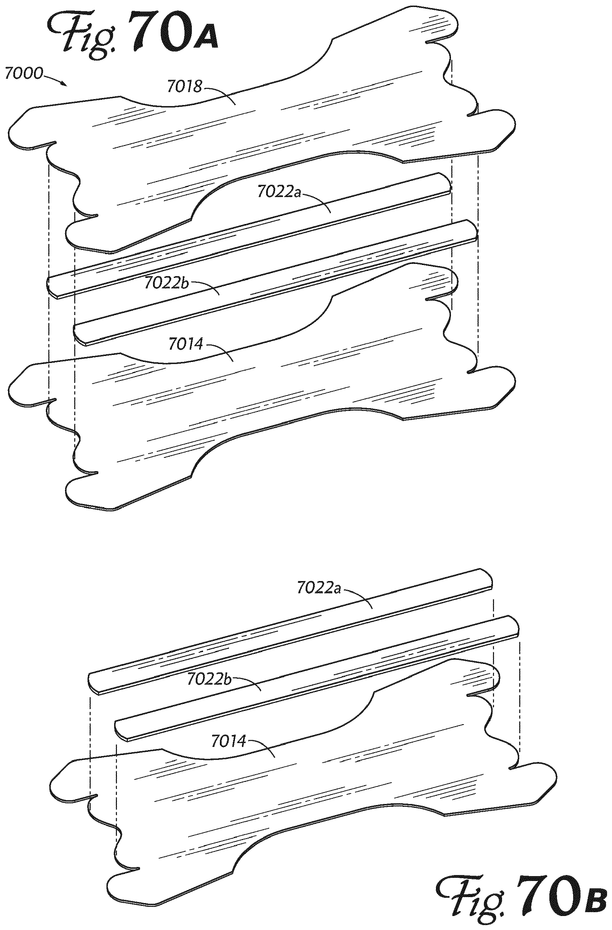

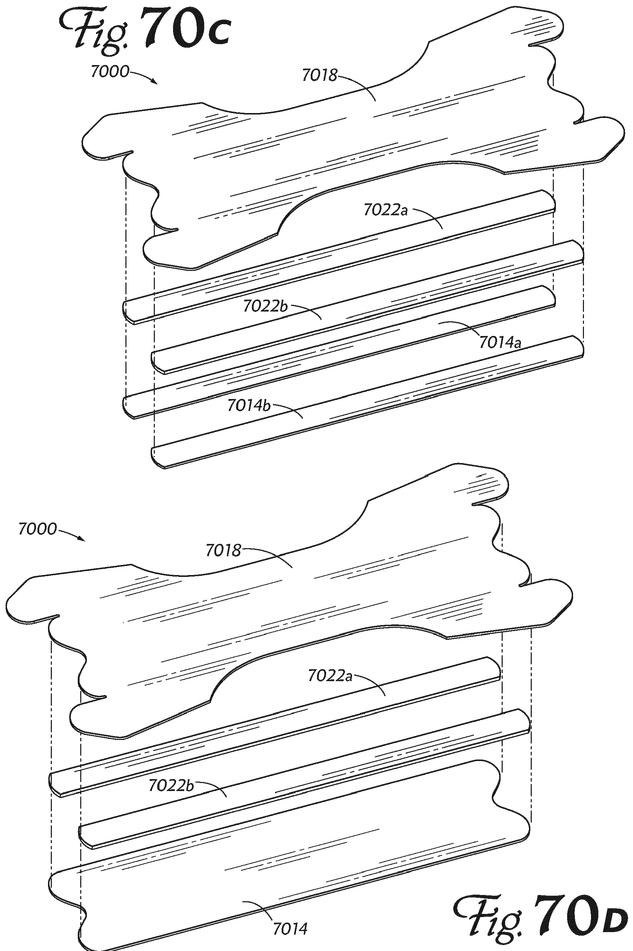

Figures 70a-70d show several different stacking orders for the materials that are laminated into a dilator according to an embodiment. Embodiments include a layer containing the resilient members (a "resilient layer"), and: 1) a cover layer over the resilient members; 2) a base layer under the resilient members; or 3) both a cover layer and a base layer. The cover and/or base layers define the outer peripheral boundary of the dilator, and are referred to generally as the "engagement layer." Those layers may be the same shape (i.e., the shape of the dilator outline), as shown in Figure 70a, 7014 and 7018 are the same shape; or a layer may be the same shape as the resilient members (Figure 70c, 7014a and 7014b are the same shape as 7022a and 7022b, respectively); or the base layer may approximate the resilient-structure bounding box (Figure 70d, base layer 7014 covers just the bounding box 7099); or the cover layer may be omitted entirely (Figure 70b only includes base layer 7014, which defines the dilator periphery). The various layer shapes and stacking configurations have ramifications in material use and ease of manufacture, as well as a modest effect on the performance of the dilator, but the exact choice of layers, layer shapes and stacking configuration are within the sound engineering judgment of one of ordinary skill, who can construct an embodiment by ensuring that the proportion of the resilient structure bounding box covered by resilient member material is less than a predetermined critical value.

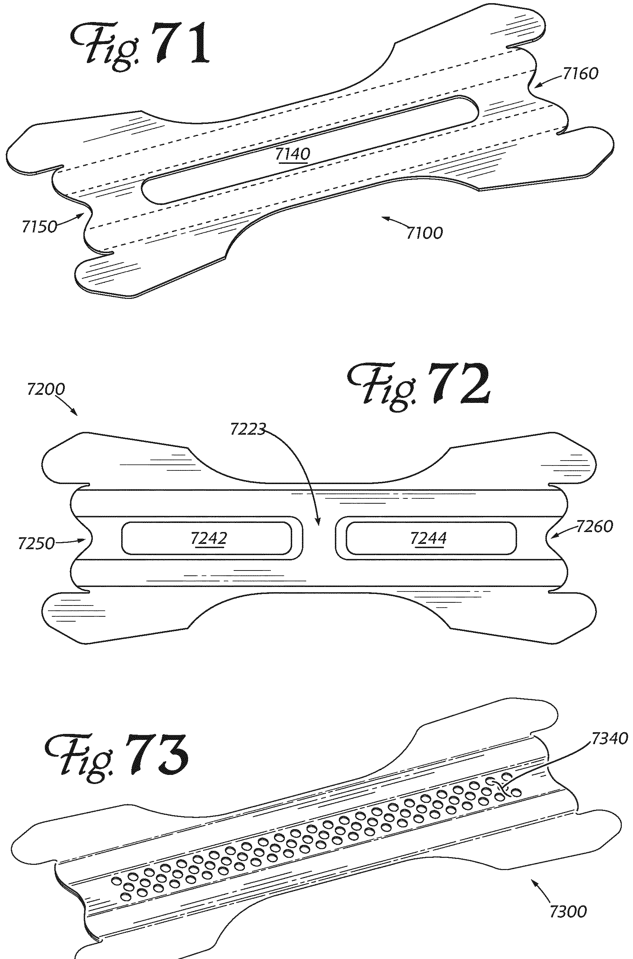

Figure 71 shows another embodiment of the invention 7100, where a central opening 7140 has been made through the base and/or cover layers (through the engagement layer), between the resilient members, and not extending all the way to the lateral ends of the dilator, so that the resilient members are still constrained, effectively connected to each other at their lateral ends via the engagement layer (7150, 7160). Opening 7140 has a higher moisture-vapor transmission rate ("MVTR") than even the permeable base and/or cover layer, so this dilator may be even more comfortable than a basic embodiment such as that shown in Figure 67.

Figure 72 shows another embodiment of the invention 7200. Here, the resilient member bands are connected by a bridge in the center of the dilator (7223), so they form an "H" shape, rather than two separate, parallel bands. Two openings 7242, 7244 expose skin on the sides of the user's nose and permit moisture to evaporate easily from the skin. This embodiment is different from the "butterfly" dilators because the ends of the resilient members - the tops and bottoms of the H shape - are still constrained: they are connected together by the base and/or cover layer (7250, 7260), whereas a butterfly dilator has resilient-member ends that can move independently.

Figure 73 shows another embodiment of the invention 7300. This embodiment is similar to Figure 67, 6700 and Figure 71, 7100, but instead of a single large opening 7140, the engagement layer between the resilient members and within the resilient structure bounding box is pierced by a plurality of openings 7340. Thus, the high-MVTR area that is all consolidated into a single opening 7140 in the embodiment of Figure 71, is spread out somewhat in the embodiment of Figure 73.

The principal distinguishing characteristic of an embodiment is the proportion of the resilient-member surface area that is covered by low-permeability or impermeable resilient members, compared to the total surface area of bounding box 6799 / central band 6737. An embodiment reduces that proportion (with a corresponding increase in the higher-permeability area of non-resilient-member engagement layer surface area), by making the resilient members slightly narrower, and by separating the resilient members by a greater distance, while maintaining substantially the same central band width and spring force resiliency as the prior art. Some embodiments increase the effective MVTR of the bounding box area by piercing or removing some of the engagement layer within that area.

Note that the nasal dilator embodiments depicted in the foregoing figures are proportioned substantially accurately, although some material thicknesses are slightly exaggerated in some views for clarity. In particular, plan-view widths, lengths, and therefore surface areas, are proportioned so that they can be directly compared by visual inspection. Numerically, an embodiment is distinguished by a resilient structure bounding box where no more than 57.5% of the total area is occupied by low-permeability or impermeable resilient members. In other words, considering the length and width of the resilient members and the gap(s) separating them, the area covered by the resilient members is 57.5% or less. The thickness of the resilient members can be adjusted to increase resiliency as necessary to attain the target resilient or spring force, measured as detailed below, of between about 15 grams and about 45 grams.

In a preferred embodiment, the area of the resilient structure bounding box covered by low-permeability or impermeable resilient members is about 47% - less than half. An embodiment may comprise even narrower (though thicker) resilient members. The practical lower limit is about 40-43% - beyond that limit, the resilient members become unreasonably thick, complicating manufacturing procedures.

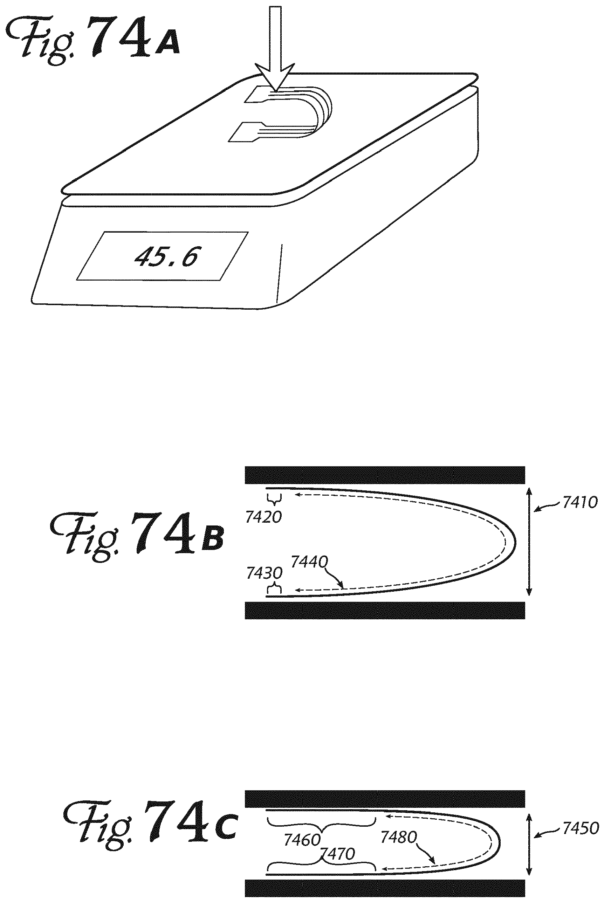

Figures 74a through 74c illustrate a protocol for measuring and comparing spring force resiliency between nasal dilators of the present invention and the prior art. As seen in Fig. 74a, a first end of the nasal dilator is forced toward its opposite end in a "U" shape, with one end placed flat against the surface of a scale. Only the very ends of the resilient members (7420, 7430) are forced to the parallel position between the scale surface and the pressing force (Fig. 74b, distance 7410), so that the resiliency of almost the full length of the resilient structure, 7440, can be measured. This arrangement yields the most accurate and repeatable readings of maximum spring force resiliency, and ensures a fair comparison between different nasal dilator devices. (It may be apparent to one of ordinary skill that if more pressure is applied to the resilient member, Fig. 74c, distance 7450, then longer portions of the resilient member structure, 7460 & 7470, are forced parallel. This shortens the length of the measured portion of the resilient member, shown at 7480, and gives an incorrect, high reading for the spring force.) This protocol is also believed to be a reasonable proxy for maximum in situ spring force resiliency, in that human noses are not typically narrower than the degree of dilator flexure caused by this measurement method.

The above disclosure may be summarized as comprising the following embodiments.
Embodiment 1: A nasal dilator having a plan view periphery and a total width, length, and surface area, comprising:
   a central oblong body region extending the total length of the nasal dilator, the body region having a width not greater than 45% of the dilator width and a body region surface area;
   an impermeable surface area contained entirely within the body region; and a permeable surface area;
   wherein a portion of the permeable surface area is contained within the body region, said portion being from about 43% to about 54% of the body region surface area; and
   wherein a portion of the permeable surface area is contained outside the body region, said portion being from about 38% to about 43% of the nasal dilator surface area.
Embodiment 2: The nasal dilator of Embodiment 1, further comprising at least one resilient member having a periphery and a width, said periphery defining the impermeable surface area.
Embodiment 3: The nasal dilator of Embodiment 2, wherein the width of the at least one resilient member is from about 45% to about 55% of the body region width; and
   wherein the surface area of the at least one resilient member is from about 46% to about 57% of the body region surface area.
Embodiment 4: The nasal dilator of Embodiment 2, wherein the width of each of the at least one resilient member is less than 0.125" and a thickness thereof is greater than 0.010".
Embodiment 5: The nasal dilator of Embodiment 2, wherein the at least one resilient member consists of two or three resilient members spaced laterally apart; and
   wherein a combined width of the two or three resilient members is from about one-fifth to about one-fourth of the nasal dilator width.
Embodiment 6: The nasal dilator of Embodiment 2, wherein the at least one resilient member consists of two resilient members; and
   wherein a combined width of the two resilient members is about 19% of the nasal dilator width.
Embodiment 7: The nasal dilator of Embodiment 2, wherein the at least one resilient member consists of two resilient members laterally spaced apart by a distance greater than a combined width of the two resilient members; and
   wherein the body region width is about 2.2 times greater than the combined width of the two resilient members.
Embodiment 8: The nasal dilator of Embodiment 2, further comprising:
   a first corner tab region abutting a first long edge of the body region; and
   a second corner tab region abutting a second long edge of the body region,
   the first and second corner tab regions having a surface area and extending the full length of the nasal dilator; and
   wherein a total surface area of the first and second corner tab regions combined is about 40% of the nasal dilator surface area, the surface area of the body region is about 60% of the nasal dilator surface area.
Embodiment 9: The nasal dilator of Embodiment 8, wherein the impermeable surface area is from about 1.6 to about 1.7 times lesser and the permeable surface area is from about 1.35 to about 1.45 times greater compared to a similar nasal dilator having a substantially same periphery and surface area, two or three resilient members positioned wholly within a same-sized body region having a surface area of about 60% of nasal dilator surface area, and a corner tab region surface area of about 40% of the nasal dilator surface area.
Embodiment 10: The nasal dilator of Embodiment 9, wherein a ratio between the permeable surface area and the impermeable surface area is from about 2:1 to about 2.5:1; and
   wherein said ratio is from about 2.3 to 2.4 times greater than the similar nasal dilator, such that the moisture vapor transmission rate of the nasal dilator is from about 36% to about 40% greater than the similar nasal dilator.
Embodiment 11: The nasal dilator of Embodiment 9, wherein the permeable body region surface area is from about 2.7 to about 3.6 times greater than the similar nasal dilator, such that a moisture vapor transmission rate of the nasal dilator is from about 36% to about 40% greater than the similar nasal dilator.
Embodiment 12: The nasal dilator of Embodiment 11, further comprising:
   at least one opening extending vertically through the body region surface area, the opening positioned adjacent the at least one resilient member,
   wherein the opening displaces a portion of the body region permeable surface area, such that the moisture vapor transmission rate of the nasal dilator is roughly 66% greater than the similar nasal dilator.
Embodiment 13: The nasal dilator of any one of Embodiments 1 to 12, wherein the engagement element comprises at least one layer selected from the group consisting of:
   i) a base member, wherein the base member is a peripheral defining layer of the nasal dilator;
   ii) a cover member, wherein the cover member is the peripheral defining layer of the nasal dilator; and
   iii) at least one base member and a cover member, wherein the cover member or the at least one base member is the peripheral defining layer of the nasal dilator.
Embodiment 14: A nasal dilator formed as a laminate comprising an engagement layer and a resilient layer, the laminate having a plan-view periphery defined by the engagement layer, wherein:
   the resilient layer comprises two adjacent, parallel resilient bands extending fully from end to end of the nasal dilator, each band having a length, width and thickness,
   a width of a gap between the two adjacent, parallel resilient bands exceeds a width of either of the two adjacent, parallel resilient bands, and
   a spring force exerted by the two adjacent, parallel resilient bands flexed in a U shape so that their ends are roughly parallel is between 15 and 45 grams.
Embodiment 15: The nasal dilator of Embodiment 14 wherein the gap between the two parallel resilient bands is constrained at at least both lateral ends of the two parallel resilient bands.
Embodiment 16: The nasal dilator of Embodiment 15 wherein the gap between the two parallel resilient bands is constrained along substantially all of a length of the gap.
Embodiment 17: The nasal dilator of Embodiment 15 wherein the resilient layer consists of the two parallel resilient bands.
Embodiment 18: The nasal dilator of Embodiment 15 wherein the gap is spanned by a portion of the engagement layer, and wherein
   the portion of the engagement layer is fully pierced by at least one opening.
Embodiment 19: The nasal dilator of Embodiment 18 wherein the at least one opening is two openings.
Embodiment 20: The nasal dilator of Embodiment 18 wherein the at least one opening is an array of openings.
Embodiment 21: A dog-bone style nasal dilator, comprising:
   a rectangular resilient truss structure; and
   an adhesive tab extension at each corner of the rectangular resilient truss structure, wherein
   a plan-view area of the rectangular resilient truss structure is divided between a resilient portion and a separate and distinct breathable portion, the resilient portion occupying no more than 57.5% of the plan-view area.
Embodiment 22: The dog-bone style nasal dilator of Embodiment 21, wherein the rectangular resilient truss structure includes a resilient member extending along one long side of the plan view area.
Embodiment 23: The dog-bone style nasal dilator of Embodiment 21, wherein the rectangular resilient truss structure includes a first resilient member extending along one long side of the plan view area, and
   a second, different resilient member extending along another long side of the plan view area.
Embodiment 24: The dog-bone style nasal dilator of Embodiment 21, wherein the resilient portion occupies no more than 47% of the plan-view area.
Embodiment 25: The dog-bone style nasal dilator of Embodiment 21, wherein the resilient portion occupies no more than 40% of the plan-view area.
Embodiment 26: A nasal dilator formed as a laminate of a plurality of layers, including a resilient layer and at least one of a base layer and a cover layer, the nasal dilator characterized in that:
   the resilient layer occupies a roughly rectangular resilient layer bounding box;
   the resilient layer comprises a first resilient member extending along one long side of the rectangular resilient layer bounding box;
   the resilient layer comprises a second resilient member extending along another long side of the rectangular resilient layer bounding box;
   the first resilient member and the second resilient member are separated by a resilient member gap; and
   an area of the resilient layer bounding box covered by the first resilient member and the second resilient member is less than 57.5% of a total area of the resilient layer bounding box.
Embodiment 27: The nasal dilator of Embodiment 26 wherein the first resilient member and the second resilient member are interconnected by a portion of the base layer or the cover layer, at least at lateral ends of the first and second resilient members.
Embodiment 28: The nasal dilator of Embodiment 26 wherein the resilient member gap is pierced by at least one opening.
Embodiment 29: The nasal dilator of Embodiment 26 wherein the resilient layer consists of the first resilient member and the second resilient member.
Embodiment 30: The nasal dilator of Embodiment 29 wherein the first resilient member and the second resilient member extend fully from end to end of the nasal dilator.

## Claims

1. A nasal dilator (7000) comprising:
a plurality of elements, wherein
at least one major surface of each of said plurality of elements is laminated to a major surface of at least one other of said plurality of elements,
said plurality of elements comprises a resilient structure and at least one of a base layer (7014) and a cover layer (7018),
said resilient structure comprises a first band portion (7022a) and a second band portion (7022b),
each of said first band portion and said second band portion extends longitudinally from a first edge of said nasal dilator to a second edge of said nasal dilator,
said second band portion is parallel to said first band portion,
said at least one of a base layer and a cover layer collectively defines an engagement layer, which engagement layer defines a plan-view periphery of said nasal dilator,
a spring force exerted by said nasal dilator in a flexed, U-shaped state in which a first portion (7420) of a major surface of said first / second band portion proximate to said first edge is parallel to a second portion (7430) of said major surface of said first / second band portion proximate to said second edge is between 15 and 45 grams,
a width of a gap between said first band portion and said second band portion exceeds a width of said first band portion, and
said width of said gap exceeds a width of said second band portion.

2. The nasal dilator of claim 1, wherein:
a width of said gap in a region proximate to said first edge is constrained by a first bridge (7150) formed between said first band portion and said second band portion by at least one of said plurality of elements other than said resilient structure,
a width of said gap in a region proximate to said second edge is constrained by a second bridge (7160) formed between said first band portion and said second band portion by at least one of said plurality of elements other than said resilient structure, and
said first bridge is distinct from said second bridge.

3. The nasal dilator of claim 2, wherein:
a width of said gap in a central region of said nasal dilator is constrained by a third bridge (7223) formed between said first band portion and said second band portion, and
said third bridge is distinct from each of said first bridge and said second bridge.

4. The nasal dilator of claim 1, wherein:
a width of said gap is constrained, from said first edge to said second edge, by a bridge formed between said first band portion and said second band portion by at least one of said plurality of elements other than said resilient structure.

5. The nasal dilator of claim 4, wherein said bridge comprises at least twenty openings (7340).

6. The nasal dilator of claim 4, wherein said bridge comprises an array of at least twenty openings (7340) having a diameter of at least 0.3 mm.

7. The nasal dilator of any one of the preceding claims, wherein:
said first portion of said first band portion and said second portion of said first band portion collectively total less than 10% of a total surface area of said first band portion, and
said first portion of said second band portion and said second portion of said second band portion collectively total less than 10% of a total surface area of said second band portion.

8. The nasal dilator of any one of the preceding claims, wherein said nasal dilator is devoid of resilient elements other than said first band portion and said second band portion.

9. A nasal dilator (6700), comprising:
at least one resilient truss structure (6722a, 6722b);
a breathable portion; and
a plurality of adhesive tab extensions, wherein
no more than 5% of a plan-view area of a rectangular bounding box (6799) enclosing an entirety of said at least one resilient truss structure falls outside an outer periphery of said nasal dilator,
a plan-view area of said nasal dilator falling within said rectangular bounding box not occupied by said at least one resilient truss structure is occupied by a first portion of said breathable portion,
a respective one of said plurality of adhesive tab extensions is located at each respective corner of said rectangular bounding box,
said at least one resilient truss structure occupies no more than a first percentage of a plan-view area of said rectangular bounding box, and
said first percentage is selected from the group consisting of 57.5%, 47% and 40%.

10. The nasal dilator of claim 9, wherein said at least one resilient truss structure comprises at least one of a first resilient member that extends along one long side of said rectangular bounding box and a second resilient member that extends along another long side of said rectangular bounding box.

11. The nasal dilator of claim 9 or 10, wherein:
said breathable portion comprises at least one opening through said nasal dilator,
said breathable portion is devoid of openings having a diameter greater than 0.3 mm other than said at least one opening,
said at least one opening occupies at least a second percentage of said plan-view area of said nasal dilator occupied by said first portion of said breathable portion, and
said second percentage is selected from the group consisting of at least 50%, at least 60%, at least 70% and at least 80%.

12. The nasal dilator of claim 11, wherein said at least one opening consists of a number of openings selected from the group consisting of 1 opening, 2 openings, 3 openings, 4 openings, 5 openings, 6 openings, 7 openings 8 openings, at least 20 openings and at least 50 openings.

13. The nasal dilator of any one of claims 9-12, wherein said rectangular bounding box encloses an entirety of said breathable portion.

14. The nasal dilator of any one of claims 9-13, wherein each of said at least one resilient truss structure extends, in a longitudinal direction of said nasal dilator, from a first outer peripheral edge of said nasal dilator to a second outer peripheral edge of said nasal dilator.

15. The nasal dilator of any one of claims 9-14, wherein said nasal dilator is a dog-bone style nasal dilator.
